# EUROPEAN PATENT SPECIFICATION

(11) **EP 3 129 446 B1**
(45) Date of publication and mention of the grant of the patent: **22.01.2025**
(21) Application number: 15777273.2
(22) Date of filing: 07.04.2015
(51) Int. Cl.: H10K 85/30, H10K 85/60, C09K 11/06, H10K 50/11

(54) **MULTI-COMPONENT HOST MATERIAL AND ORGANIC ELECTROLUMINESCENT DEVICE COMPRISING THE SAME**
MEHRKOMPONENTIGES HOST-MATERIAL UND ORGANISCHE ELEKTROLUMINESZENZVORRICHTUNG DAMIT
MATÉRIAU HÔTE À PLUSIEURS COMPOSANTS ET DISPOSITIF ÉLECTROLUMINESCENT ORGANIQUE LE COMPRENANT

(30) Priority: 08.04.2014 KR 20140041844; 10.07.2014 KR 20140086754; 26.03.2015 KR 20150042704
(43) Date of publication of application: 15.02.2017
(60) Divisional application: 24199330.2
(73) Proprietor: DuPont Specialty Materials Korea Ltd., Cheonan-si, Chungcheongnam-do (KR)
(72) Inventor: AHN, Hee-Choon, Suwon-si, Gyeonggi-do 443-400 (KR); KIM, Young-Kwang, Cheonan-si, Chungcheongnam-do 331-980 (KR); MOON, Doo-Hyeon, Hwaseong-si, Gyeonggi-do 445-768 (KR); LEE, Su-Hyun, Suwon-si, Gyeonggi-do 440-200 (KR); LEE, Seon-Woo, Cheonan-si, Chungcheongnam-do 331-980 (KR); KIM, Chi-Sik, Hwaseong-si, Gyeonggi-do 445-752 (KR); PARK, Kyoung-Jin, Seongnam-si, Gyeonggi-do 462-838 (KR); KIM, Nam-Kyun, Yongin-si, Gyeonggi-do 448-527 (KR); CHOI, Kyung-Hoon, Hwaseong-si, Gyeonggi-do 445-160 (KR); SHIM, Jae-Hoon, Cheonan-si, Chungcheongnam-do 331-980 (KR); CHO, Young-Jun, Seongnam-si, Gyeonggi-do 463-400 (KR); LEE, Kyung-Joo, Seoul 121-773 (KR)
(74) Representative: Houghton, Mark Phillip
(86) International application number: PCT/KR2015/003485
(87) International publication number: WO 2015/156587

(56) References cited:
- EP-A2- 2 821 459
- WO-A1-2011/155507
- WO-A1-2012/176818
- WO-A1-2013/058343
- WO-A1-2013/062075
- WO-A1-2013/084885
- WO-A1-2013/112557
- WO-A1-2013/147205
- WO-A1-2013/187896
- WO-A1-2014/038677
- WO-A1-2014/087913
- WO-A2-2014/096961
- JP-A- 2014 049 539
- US-A1- 2011 278 555
- US-A1- 2013 234 119
- US-A1- 2014 042 469
- US-A1- 2014 070 204
- US-A1- 2014 151 647
- US-A1- 2014 217 378
- US-A1- 2014 306 207

## Description

### Technical Field

The present invention relates to a multi-component host material and an organic electroluminescent device comprising the same.

### Background Art

An electroluminescent (EL) device is a self-light-emitting device with the advantage of providing a wider viewing angle, a greater contrast ratio, and a faster response time. An organic EL device was first developed by Eastman Kodak, by using small aromatic diamine molecules and aluminum complexes as materials for forming a light-emitting layer (see Appl. Phys. Lett. 51, 913, 1987).

The organic EL device changes electric energy into light by the injection of a charge into an organic light-emitting material and commonly comprises an anode, a cathode, and an organic layer formed between the two electrodes. The organic layer of the organic EL device may be composed of a hole injection layer (HIL), a hole transport layer (HTL), an electron blocking layer (EBL), a light-emitting layer (EML) (containing host and dopant materials), an electron buffer layer, a hole blocking layer (HBL), an electron transport layer (ETL), an electron injection layer (EIL), etc.; the materials used in the organic layer can be classified into a hole injection material, a hole transport material, an electron blocking material, a light-emitting material, an electron buffer material, a hole blocking material, an electron transport material, an electron injection material, etc., depending on functions. In the organic EL device, holes from an anode and electrons from a cathode are injected into a light-emitting layer by the injection of a charge, and an exciton having high energy is produced by the recombination of holes and electrons. The organic light-emitting compound moves into an excited state by the energy and emits light which changes from energy when the organic light-emitting compound returns to the ground state from the excited state.

The most important factor determining luminescent efficiency in an organic EL device is the light-emitting material. The light-emitting material is required to have the following features: high quantum efficiency, high movement degree of an electron and a hole, formability of a uniform layer, and stability. The light-emitting material is classified into blue light-emitting materials, green light-emitting materials, and red light-emitting materials according to the light-emitting color, and further includes yellow light-emitting materials or orange light-emitting materials. Furthermore, the light-emitting material is classified into a host material and a dopant material in the functional aspect. Recently, an urgent task is the development of an organic EL device having high efficacy and long operating lifespan. In particular, the development of highly excellent light-emitting material over conventional light-emitting materials is urgent considering EL properties required in medium- and large-sized OLED panels. For this, preferably, as a solvent in a solid state and energy transmitter, a host material should have high purity and suitable molecular weight in order to be deposited under vacuum. Furthermore, a host material is required to have high glass transition temperature and pyrolysis temperature to guarantee thermal stability, high electrochemical stability to provide long lifespan, easy formability of an amorphous thin film, good adhesion with adjacent layers, and no movement between layers.

A mixed system of a dopant/host material can be used as a light-emitting material to improve color purity, luminescent efficiency, and stability. Generally, the device having the most excellent EL properties comprises the light-emitting layer, wherein a dopant is doped onto a host. If the dopant/host material system is used, the selection of the host material is important since the host material greatly influences on efficiency and performance of a light-emitting device.

WO 2013/168688 A1, Japanese Patent No. 3139321, Korean Patent No. 10-1170666, Korean Patent Application Laying-open No. 10-2012-0013173, and WO 2013/112557 A1 disclose organic EL devices comprising a dopant/host material system. The above literature use one host component having a carbazole-carbazole skeleton or exclude a host having a cabazole skeleton from second and third hosts.

The present inventors have found that an organic EL device using a multi-component host compounds having a specific bicarbazole derivative which contains an aryl group and a specific carbazole derivative which includes a nitrogen-containing heteroaryl group has high efficiency and long lifespan, compared with using one component host compound in a light-emitting layer.

The object of the present invention is to provide an organic EL device having high efficiency and long lifespan.

### Solution to Problems

The present invention in its various aspects is as set out in the appended claims.

The above objective can be achieved by an organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by the following formula 1 which is a specific bicarbazole derivative containing an aryl group, and a second host compound is represented by the following formula 2 which is a specific carbazole derivative including a nitrogen-containing heteroaryl group: 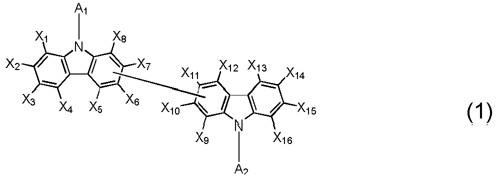 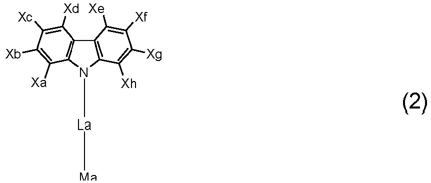 wherein
A₁ represents unsubstituted phenyl, unsubstituted biphenyl;
A₂ represents unsubstituted phenyl or unsubstituted biphenyl;
X₁ to X₁₆ each independently represent hydrogen, deuterium, a substituted or unsubstituted (C6-C60)aryl group, a substituted or unsubstituted 3- to 30-membered heteroaryl group;
Ma represents a substituted or unsubstituted triazinyl;
La represents a substituted or unsubstituted (C6-C30)arylene group;
and as further defined in claim 1.

### Effects of the Invention

According to the present invention, the organic EL device having high efficiency and long lifespan is provided and the production of a display device or a lighting device is possible by using the organic EL device.

### Embodiments of the Invention

Hereinafter, the present invention will be described in detail. However, the following description is intended to explain the invention, and is not meant in any way to restrict the scope of the invention.

The invention is defined by claim 1.

In formula 2, La represents a substituted or unsubstituted (C6-C30)arylene group such as selected from the following formulas 10 to 19: 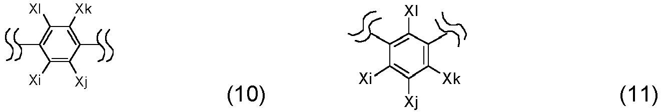 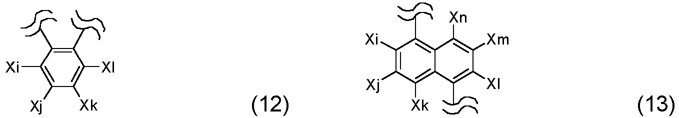 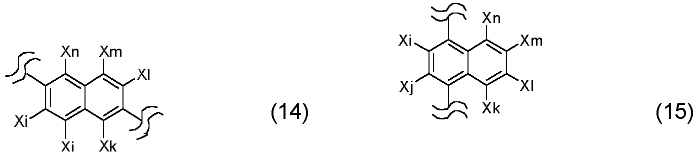 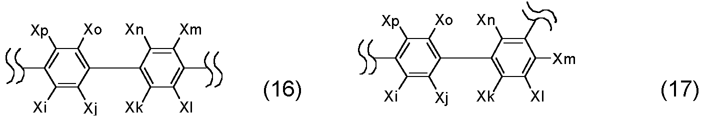 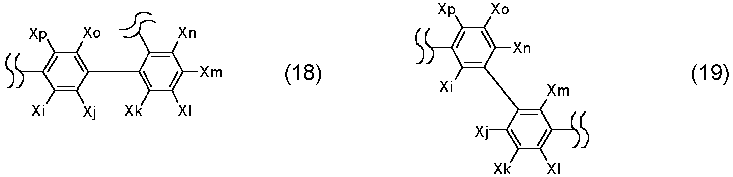 wherein
Xi to Xp each independently represent hydrogen, deuterium.

In formula 2, Ma represents triazinyl.

Herein, "(C1-C30)alkyl(ene)" is meant to be a linear or branched alkyl(ene) having 1 to 30 carbon atoms, in which the number of carbon atoms is preferably 1 to 20, more preferably 1 to 10, and includes methyl, ethyl, n-propyl, isopropyl, n-butyl, isobutyl, tert-butyl, etc. "(C2-C30)alkenyl" is meant to be a linear or branched alkenyl having 2 to 30 carbon atoms, in which the number of carbon atoms is preferably 2 to 20, more preferably 2 to 10, and includes vinyl, 1-propenyl, 2-propenyl, 1-butenyl, 2-butenyl, 3-butenyl, 2-methylbut-2-enyl, etc. "(C2-C30)alkynyl" is a linear or branched alkynyl having 2 to 30 carbon atoms, in which the number of carbon atoms is preferably 2 to 20, more preferably 2 to 10, and includes ethynyl, 1-propynyl, 2-propynyl, 1-butynyl, 2-butynyl, 3-butynyl, 1-methylpent-2-ynyl, etc. "(C3-C30)cycloalkyl" is a mono- or polycyclic hydrocarbon having 3 to 30 carbon atoms, in which the number of carbon atoms is preferably 3 to 20, more preferably 3 to 7, and includes cyclopropyl, cyclobutyl, cyclopentyl, cyclohexyl, etc. "3- to 7-membered heterocycloalkyl" is a cycloalkyl having at least one heteroatom selected from the group consisting of B, N, O, S, P(=O), Si and P, preferably O, S and N, and 3 to 7, preferably 5 to 7 ring backbone atoms, and includes tetrahydrofuran, pyrrolidine, thiolan, tetrahydropyran, etc. "(C6-C30)aryl(ene)" is a monocyclic or fused ring derived from an aromatic hydrocarbon having 6 to 30 carbon atoms, in which the number of carbon atoms is preferably 6 to 20, more preferably 6 to 15, and includes phenyl, biphenyl, terphenyl, naphthyl, fluorenyl, phenanthrenyl, anthracenyl, indenyl, triphenylenyl, pyrenyl, tetracenyl, perylenyl, chrysenyl, naphthacenyl, fluoranthenyl, etc. "3- to 30-membered heteroaryl(ene)" is an aryl group having at least one, preferably 1 to 4 heteroatom selected from the group consisting of B, N, O, S, P(=O), Si and P, and 3 to 30 ring backbone atoms; is a monocyclic ring, or a fused ring condensed with at least one benzene ring; has preferably 3 to 20, more preferably 3 to 15 ring backbone atoms; may be partially saturated; may be one formed by linking at least one heteroaryl or aryl group to a heteroaryl group via a single bond(s); and includes a monocyclic ring-type heteroaryl including furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., and a fused ring-type heteroaryl including benzofuranyl, benzothiophenyl, isobenzofuranyl, anyl, dibenzothiophenyl, benzoimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenoxazinyl, phenanthridinyl, benzodioxolyl, etc. "Nitrogen-containing 5- to 30-membered heteroaryl(ene) group" is an aryl(ene) group having at least one heteroatom N and 5 to 30 ring backbone atoms. 5 to 20 ring backbone atoms and 1 to 4 heteroatom are preferable, and 5 to 15 ring backbone atoms are more preferable. It is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including pyrrolyl, imidazolyl, pyrazolyl, triazinyl, tetrazinyl, triazolyl, tetrazolyl, pyridyl, pyrazinyl, pyrimidinyl, pyridazinyl, etc., and a fused ring-type heteroaryl including benzoimidazolyl, isoindolyl, indolyl, indazolyl, benzothiadiazolyl, quinolyl, isoquinolyl, cinnolinyl, quinazolinyl, quinoxalinyl, carbazolyl, phenanthridinyl, etc. "Halogen" includes F, Cl, Br and I.

Herein, "substituted" in the expression "substituted or unsubstituted" means that a hydrogen atom in a certain functional group is replaced with another atom or group, i.e., a substituent. Substituents of the substituted alkyl(ene) group, the substituted alkenyl group, the substituted alkynyl group, the substituted cycloalkyl group, the substituted aryl(ene) group, the substituted heteroaryl(ene) group, the substituted trialkylsilyl group, the substituted triarylsilyl group, the substituted dialkylarylsilyl group, the substituted mono- or di-arylamino group, or the substituted mono- or polycyclic, (C3-C30) alicyclic or aromatic ring are each independently at least one selected from the group consisting of deuterium; a halogen; a cyano group; a carboxyl group; a nitro group; a hydroxyl group; a (C1-C30)alkyl group; a halo(C1-C30)alkyl group; a (C2-C30)alkenyl group; a (C2-C30)alkynyl group; a (C1-C30)alkoxy group; a (C1-C30)alkylthio group; a (C3-C30)cycloalkyl group; a (C3-C30)cycloalkenyl group; a 3- to 7-membered heterocycloalkyl group; a (C6-C30)aryloxy group; a (C6-C30)arylthio group; a 3- to 30-membered heteroaryl group which is unsubstituted or substituted with a (C6-C30)aryl group; a (C6-C30)aryl group which is unsubstituted or substituted with a cyano group, a 3- to 30-membered heteroaryl group, or a tri(C6-C30)arylsilyl group; a tri(C1-C30)alkylsilyl group; a tri(C6-C30)arylsilyl group; a di(C1-C30)alkyl(C6-C30)arylsilyl group; a (C1-C30)alkyldi(C6-C30)arylsilyl group; an amino group; a mono- or di(C1-C30)alkylamino group; a mono- or di(C6-C30)arylamino group; a (C1-C30)alkyl(C6-C30)arylamino group; a (C1-C30)alkylcarbonyl group; a (C1-C30)alkoxycarbonyl group; a (C6-C30)arylcarbonyl group; a di(C6-C30)arylboronyl group; a di(C1-C30)alkylboronyl group; a (C1-C30)alkyl(C6-C30)arylboronyl group; a (C6-C30)aryl(C1-C30)alkyl group; and a (C1-C30)alkyl(C6-C30)aryl group. Preferably, the substituents are each independently at least one selected from the group consisting of
a (C1-C6)alkyl group; a 5- to 15-membered heteroaryl group; a (C6-C18)aryl group which is unsubstituted or substituted with a cyano group or a tri(C6-C12)arylsilyl group; a tri(C6-C12)arylsilyl group; and a (C1-C6)alkyl(C6-C12)aryl group.

The compound of formula 1 as a first host compound may be selected from the group consisting of following listed compounds, except compounds in the list which are not within the scope of the present invention are H1-3 to H1-6, H1-10, H1-11, H1-13, H1-21 to H1-24, H1-26 to H1-31, H1-34, H1-38, H1-40, H1-49, H1-73 to H1-96, H1-113 to H1-122, H1-124, H1-126 to H1-189 and H1-291 to H1-296.

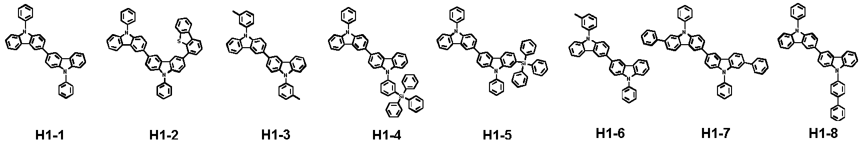

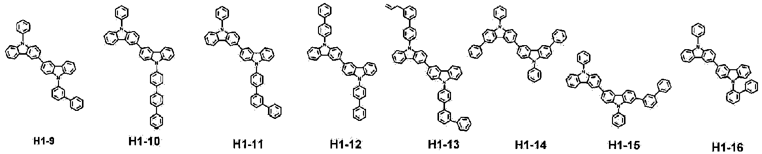

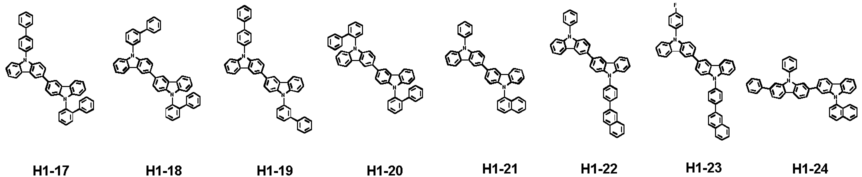

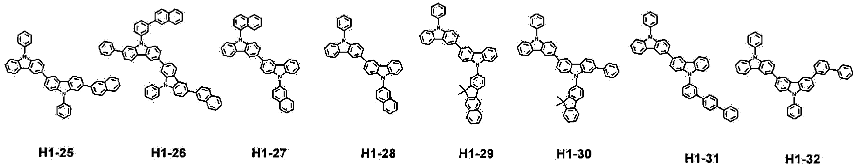

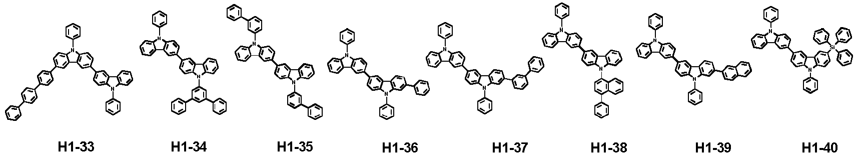

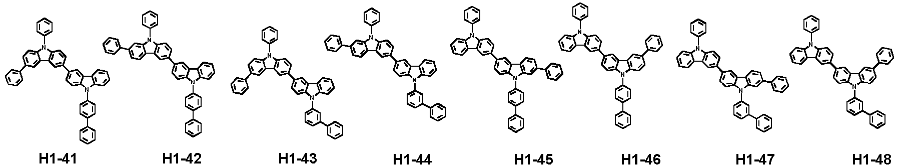

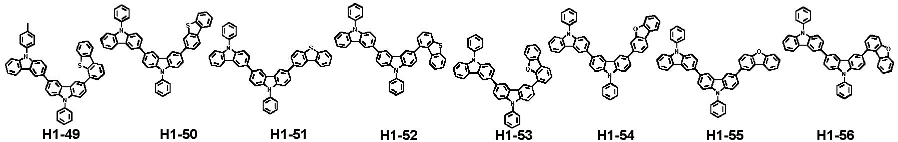

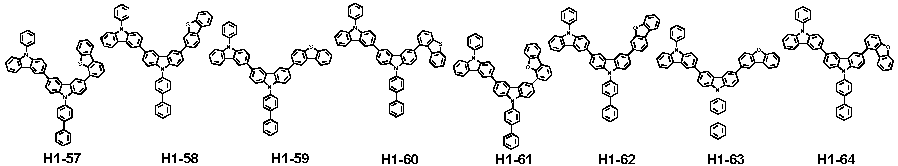

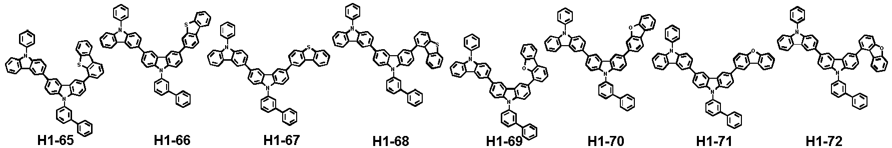

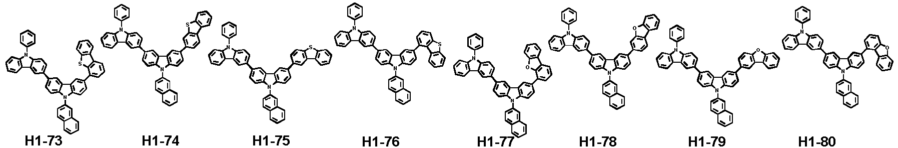

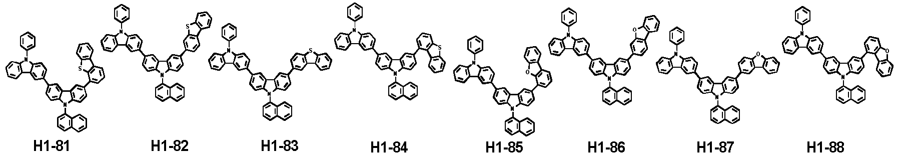

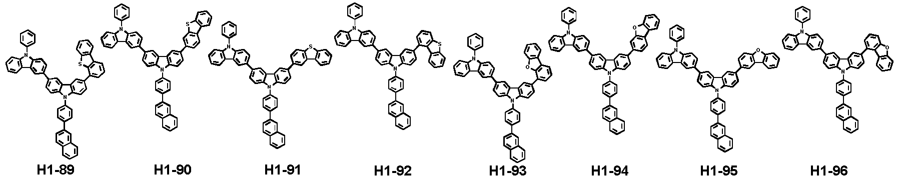

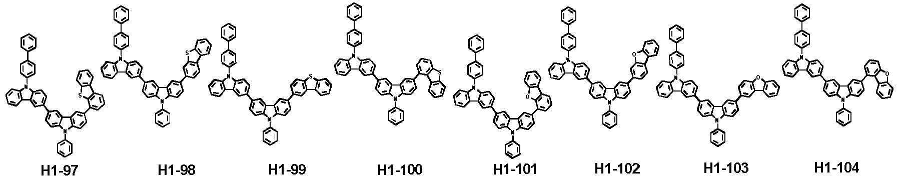

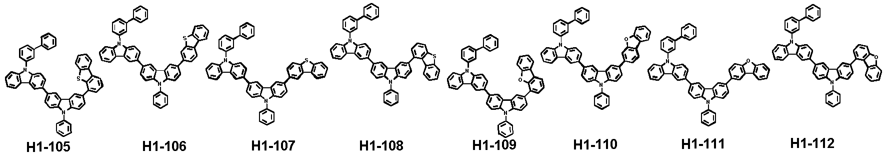

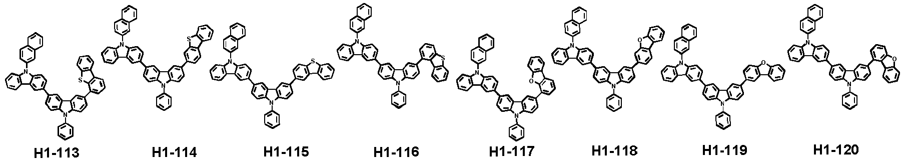

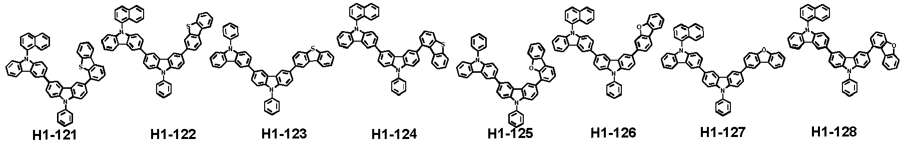

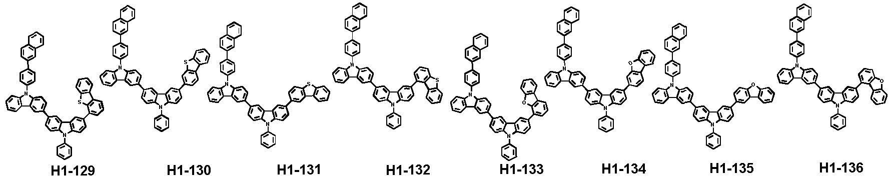

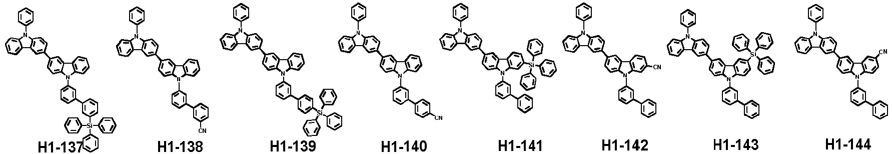

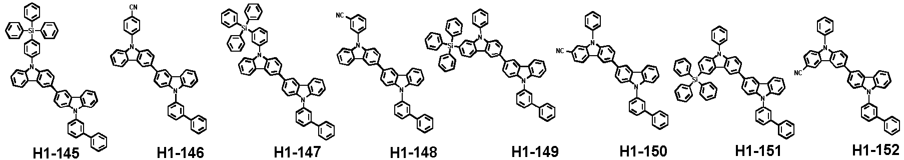

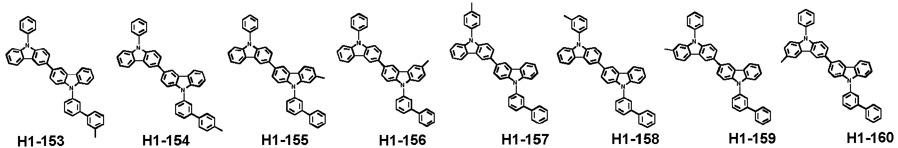

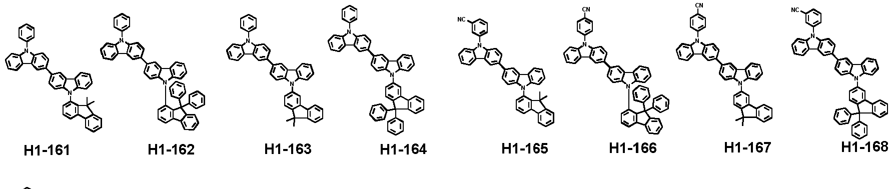

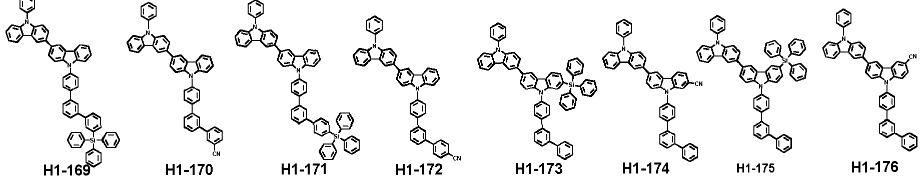

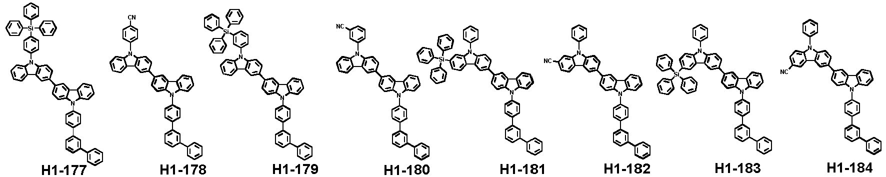

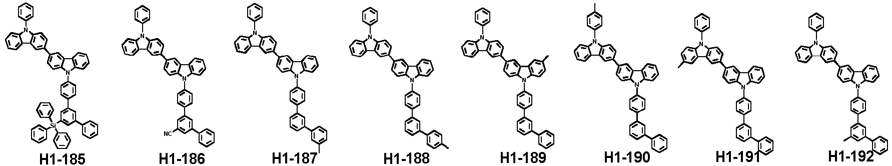

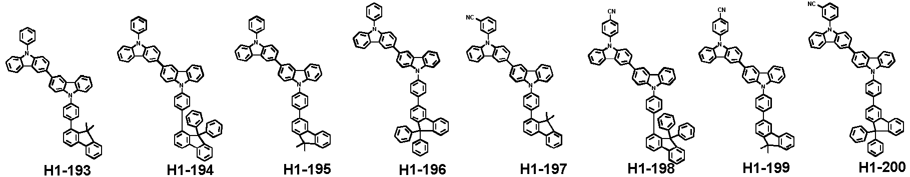

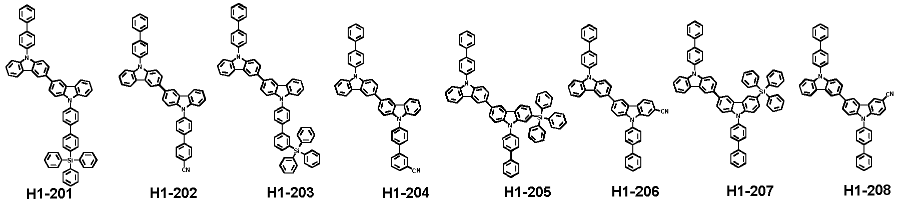

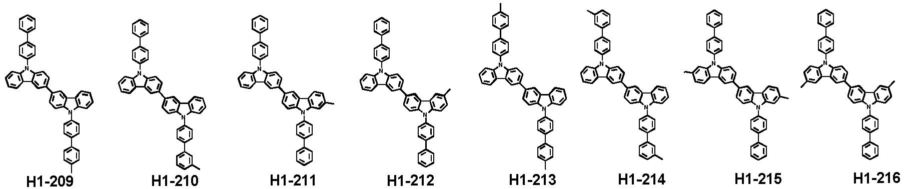

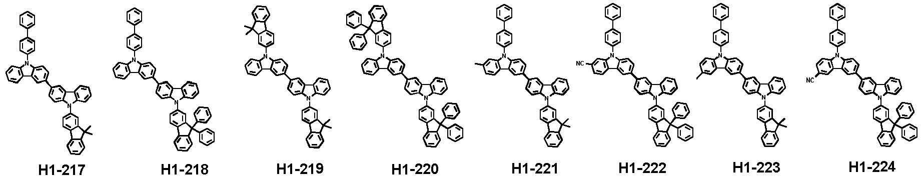

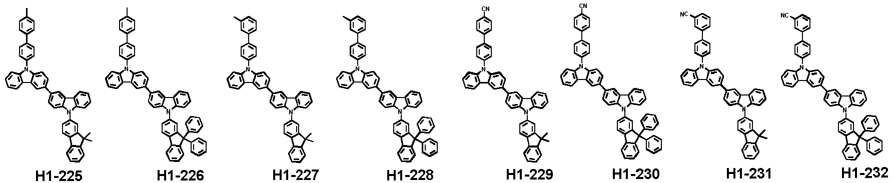

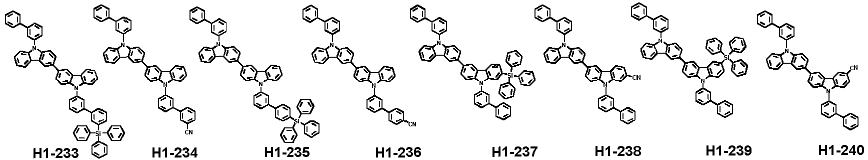

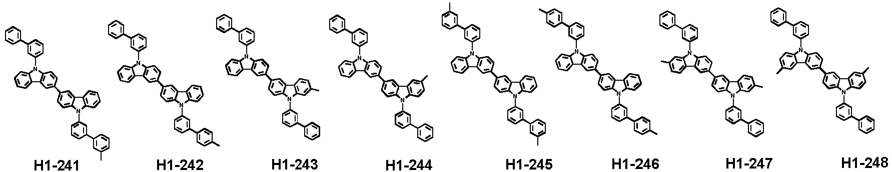

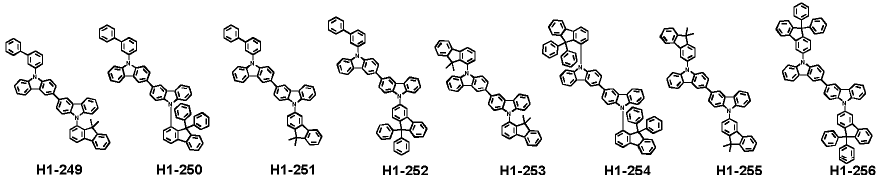

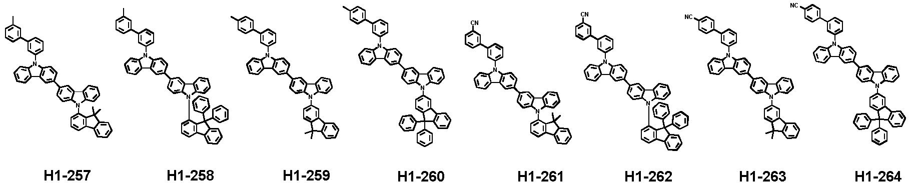

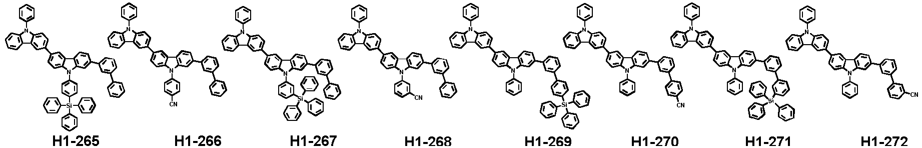

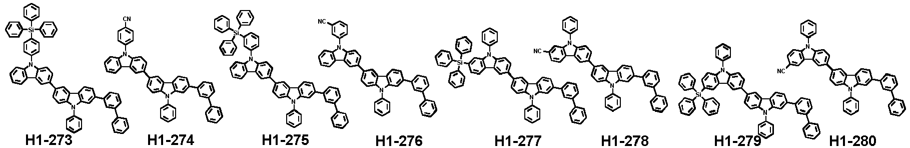

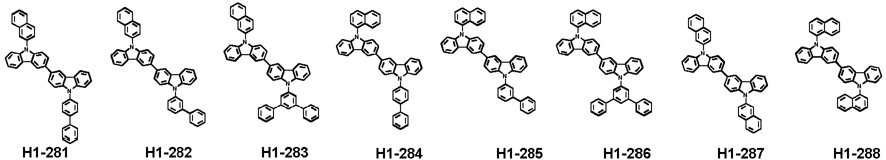

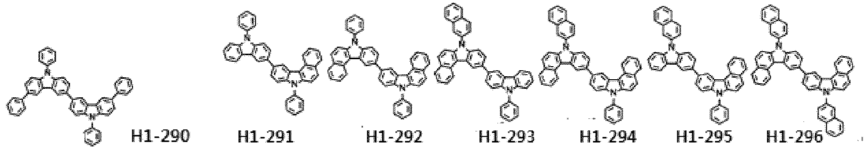

The compound of formula 2 as a second host compound may be selected from the group consisting of following compounds: except compounds in the list which are not within the scope of the present invention are H2-1 to H2-100, H2-103, H2-104, H2-106 to H2-110, H2-113, H2-114, H2-116 to H2-122, H2-124, H2-126 to H2-139, H2-144, H2-149, H2- 154 to H2-160, H2-162, H2-164, H2-165, H2-168, H2-170, H2-172, H2-173, H2-177 to H2-182, H2-184, H2-186 to H2-257, H2-261 to H2-271, H2-275 to H2-286, H2-291 to H2-302, H2-307 to H2-318, H2-320, H2-322 to H2-334, H2-336, H2-338, H2-344 to H2-346, H2-348 to H2-351, H2-355, H2-358, H2-359, H2-361 to H2-365, H2-367, H2-371, H2-374, H2-375, H2-377 to H2-381, H2-383, H2-387, H2-390, H2-391, H2-393 to H2-397, H2-399, H2-403, H2-406, H2-407, H2-409 to H2-413, H2-415, H2-419, H2-423, H2-426, H2-427, H2-429 to H2-433, H2-435, H2-439, H2-442, H2-443, H2-445 to H2-449, H2-451, H2-455, H2-458, H2-459, H2-461 to H2-465, H2-467, H2-471 to H2-475, H2-477, H2-478, H2-481 to H2-484, H2-493 to H2-495, H2-533 to H2-607 and H2-617 to H2-671. H2-2612H2-

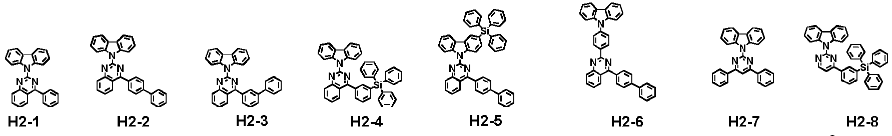

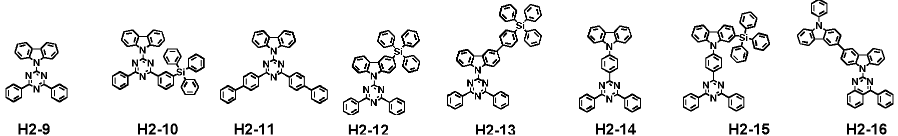

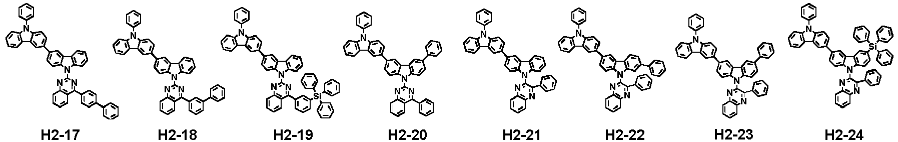

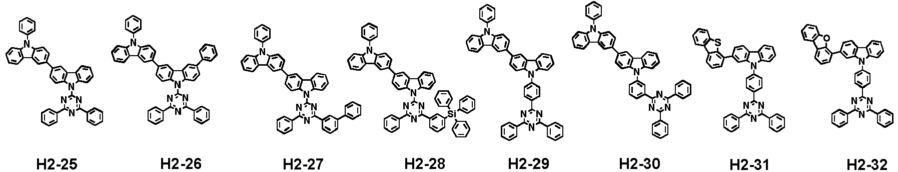

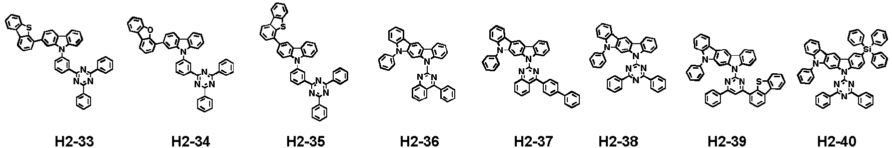

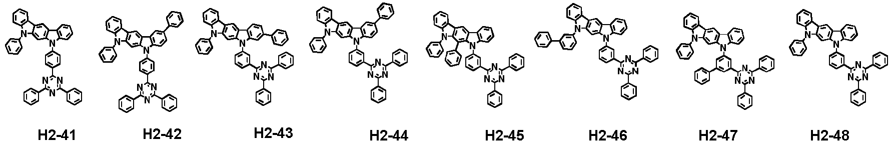

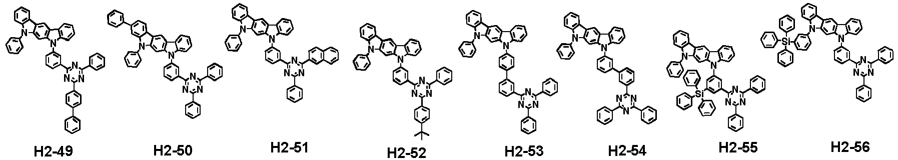

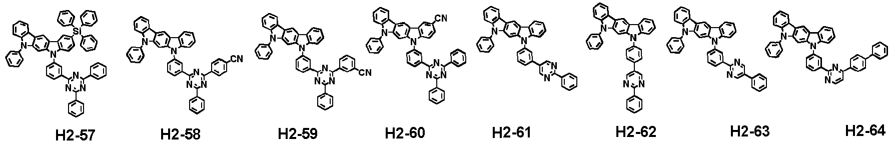

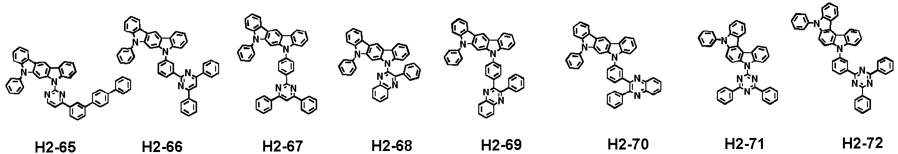

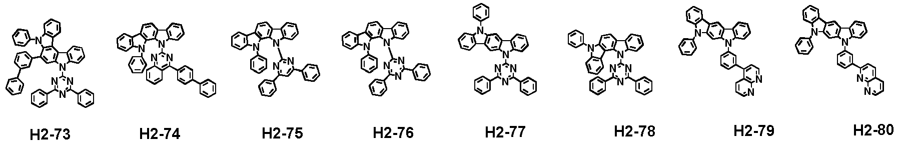

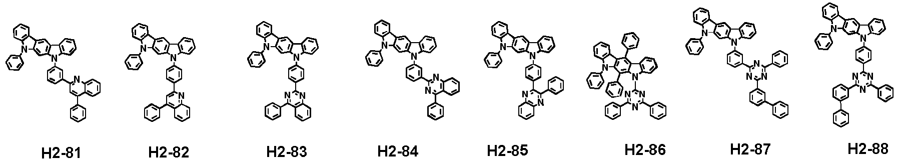

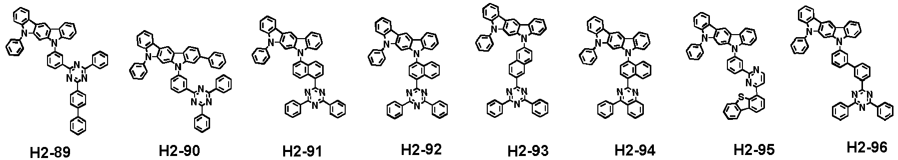

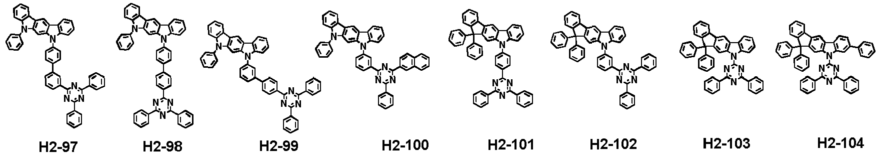

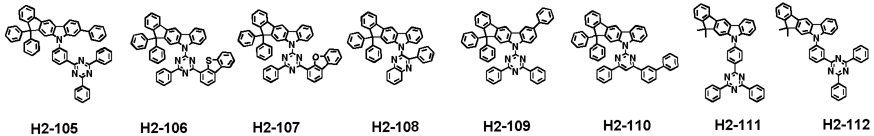

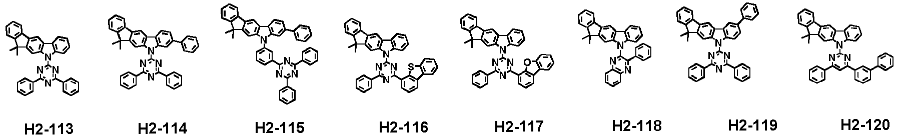

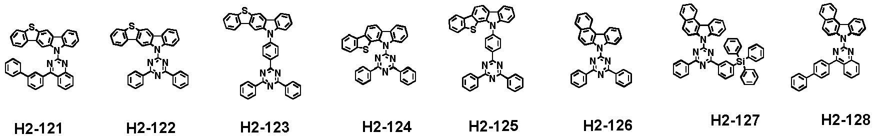

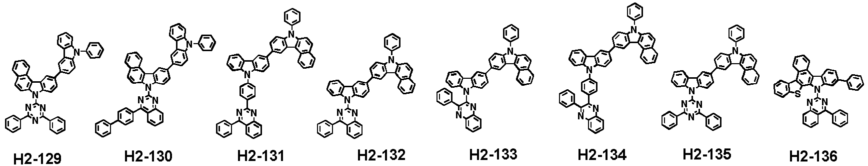

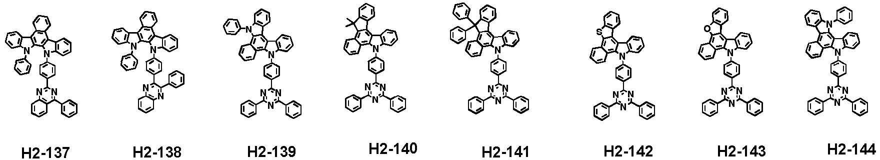

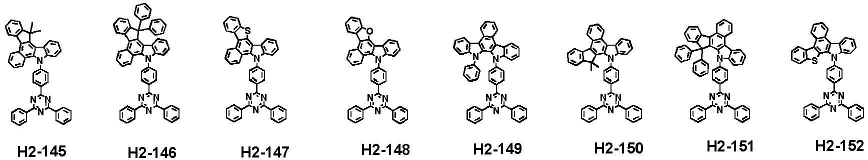

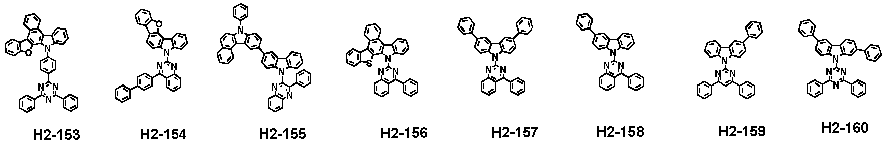

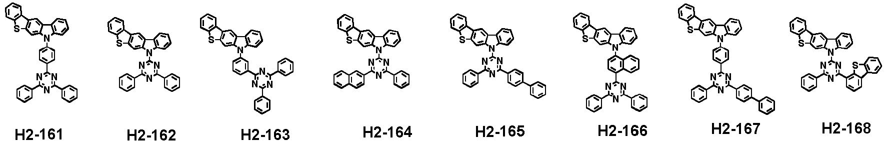

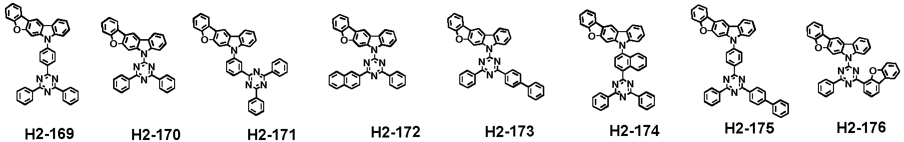

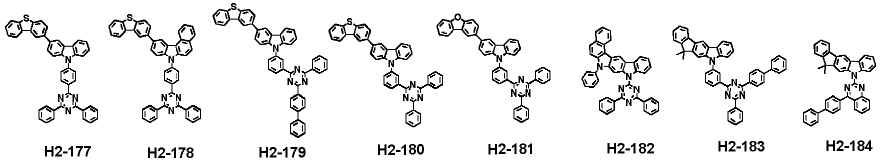

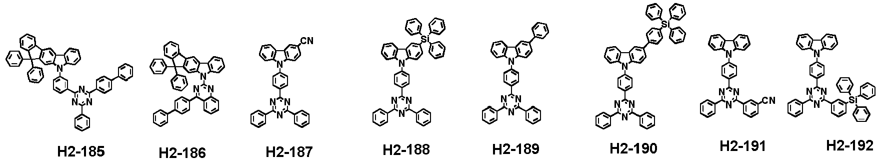

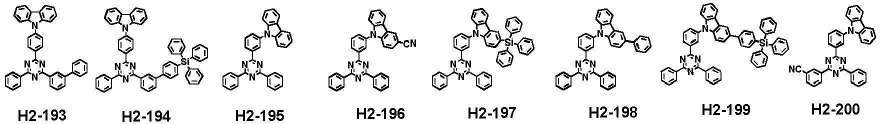

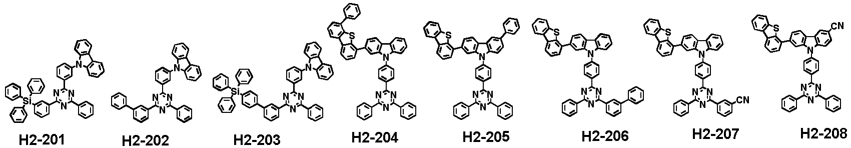

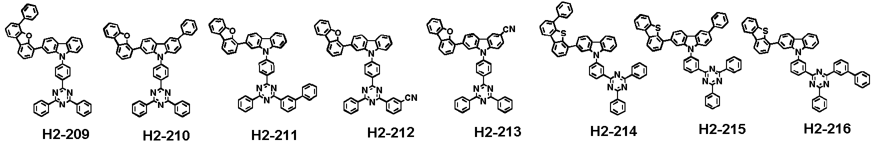

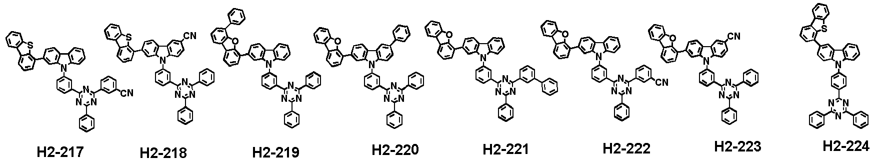

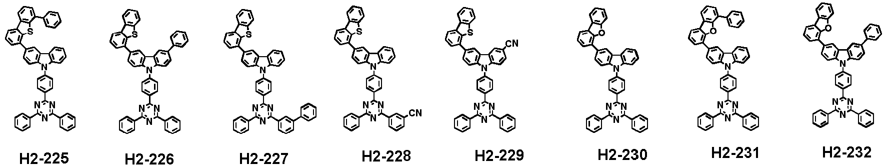

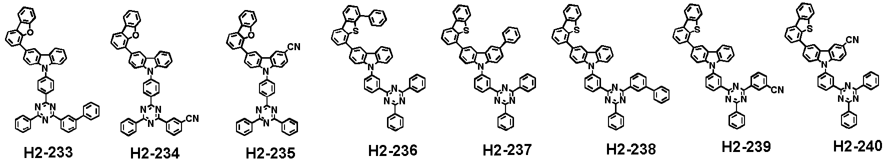

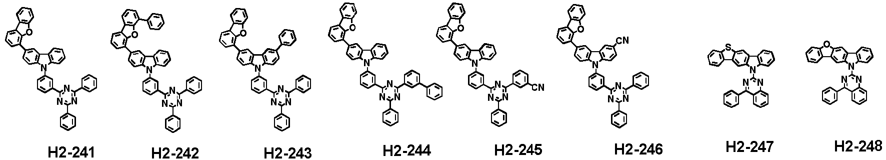

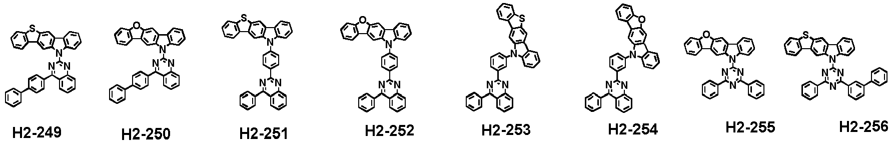

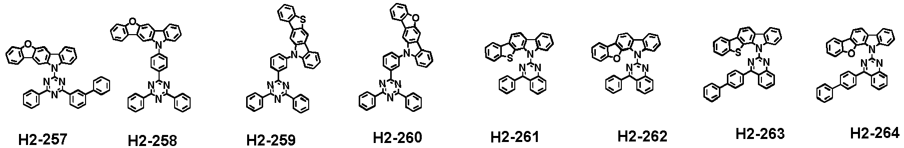

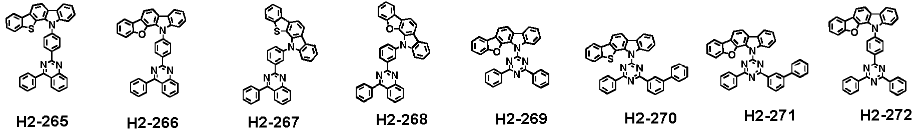

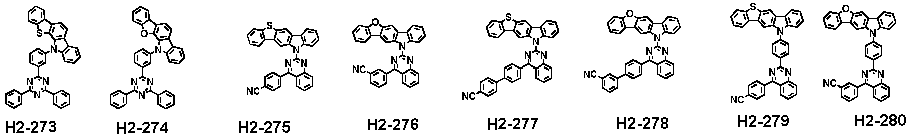

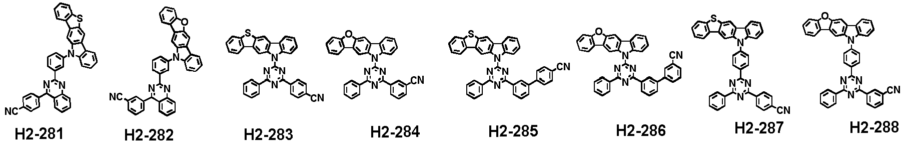

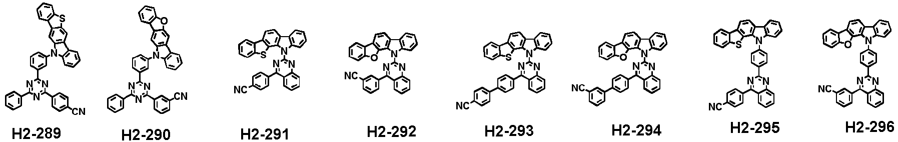

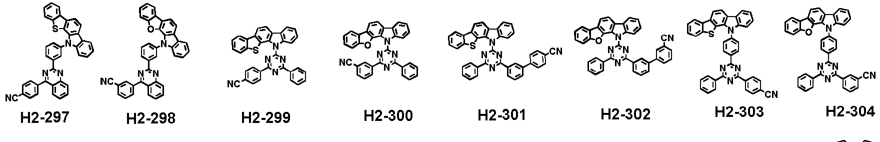

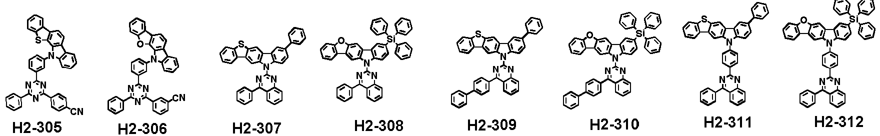

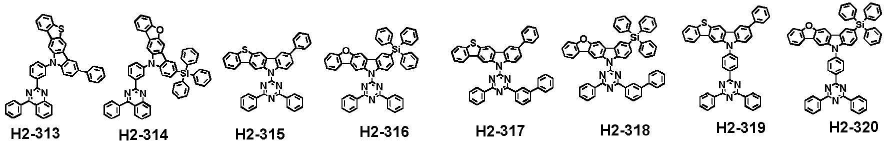

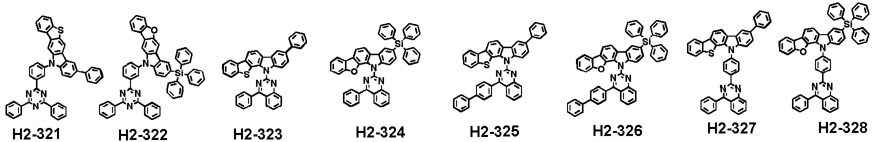

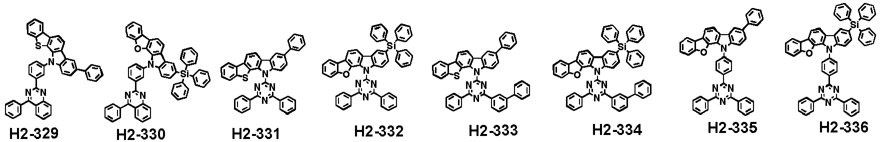

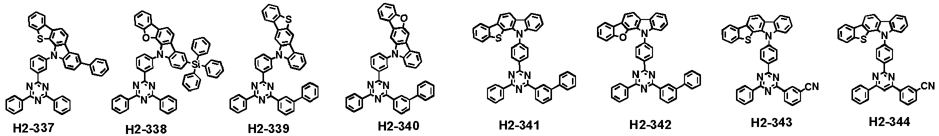

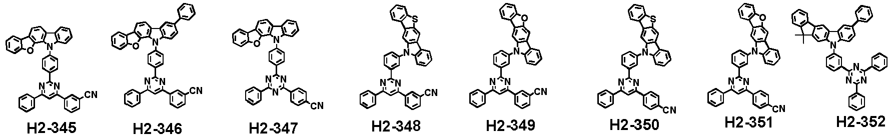

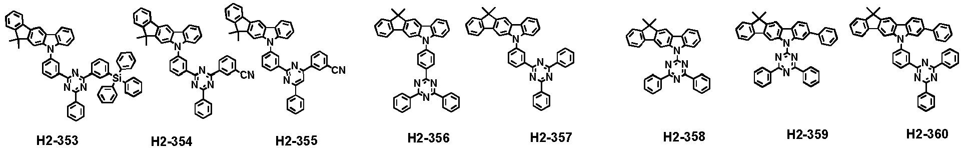

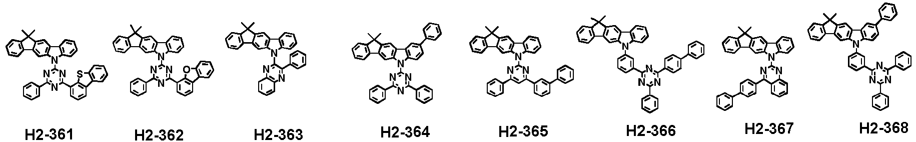

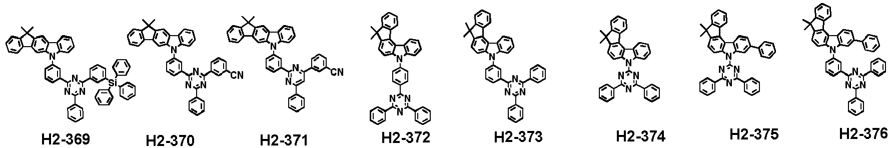

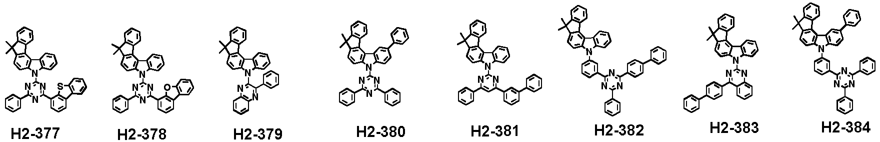

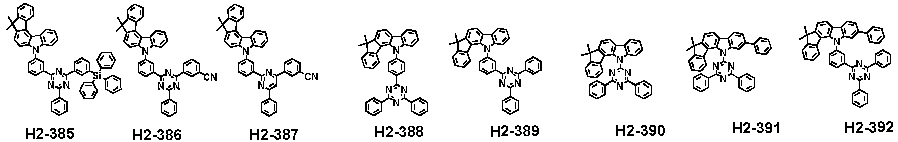

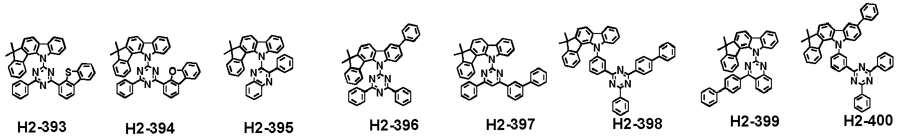

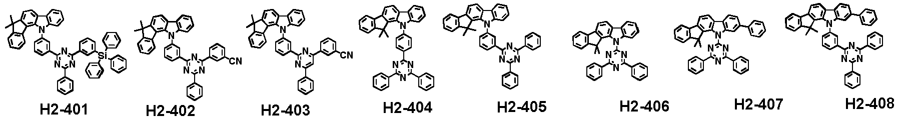

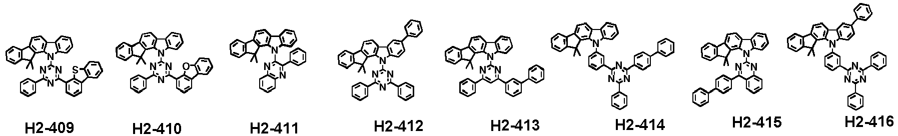

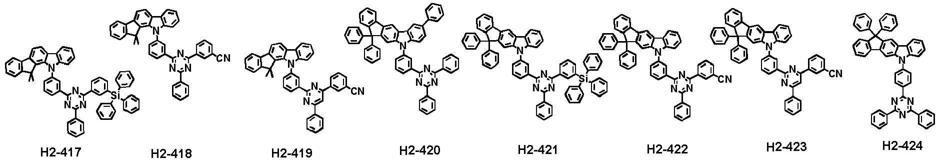

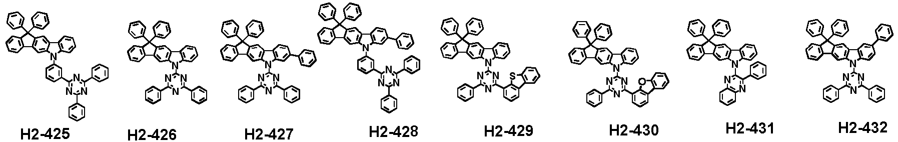

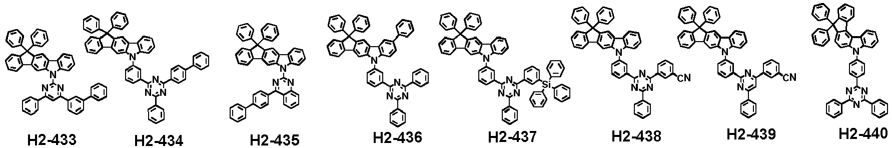

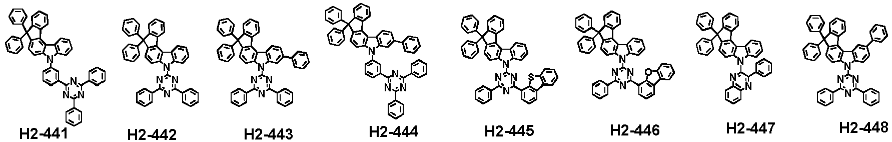

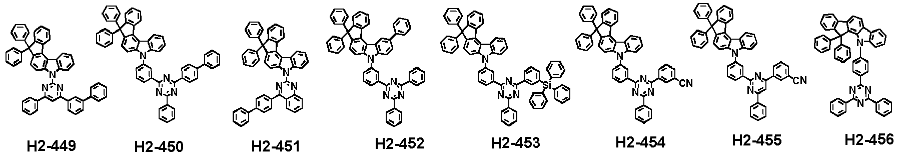

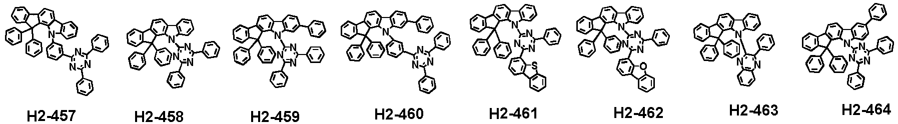

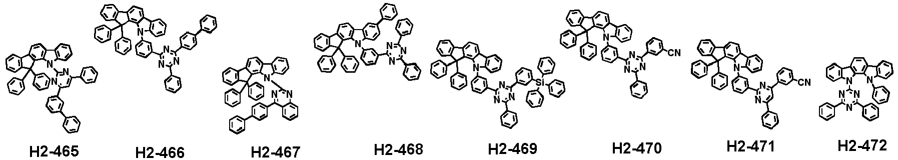

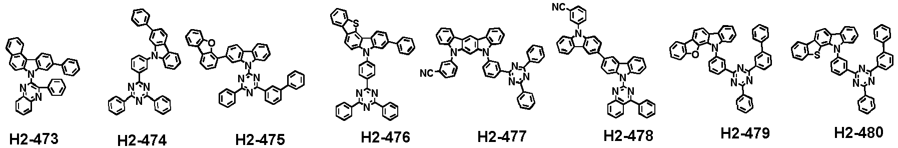

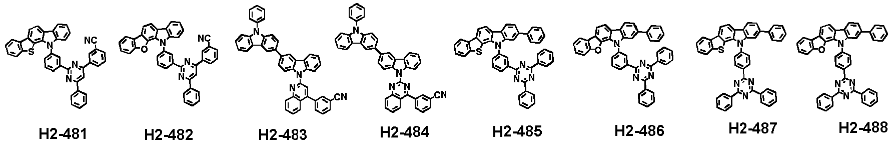

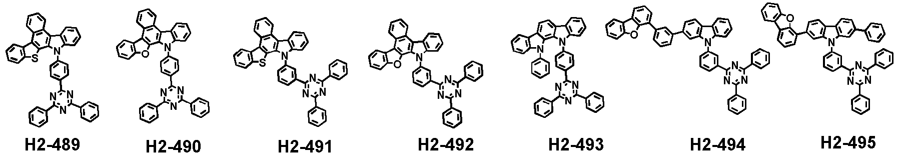

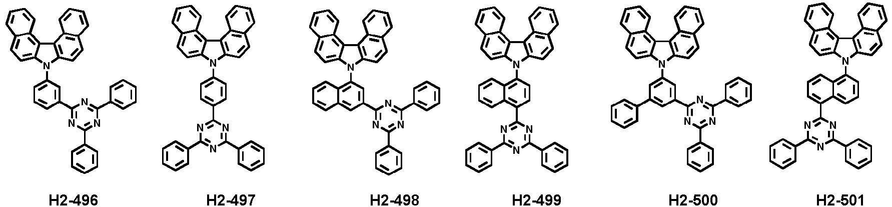

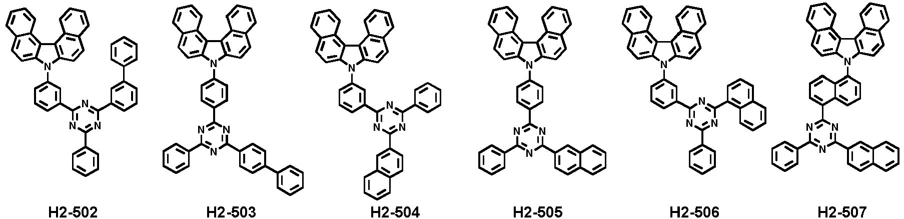

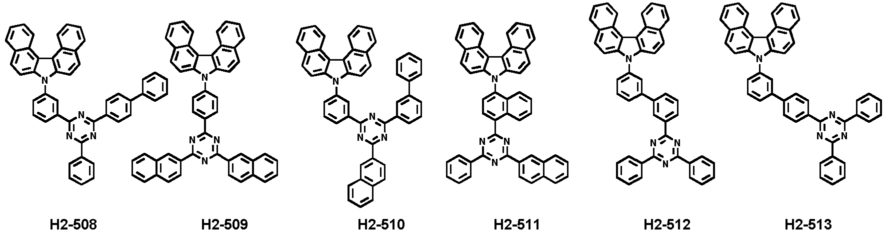

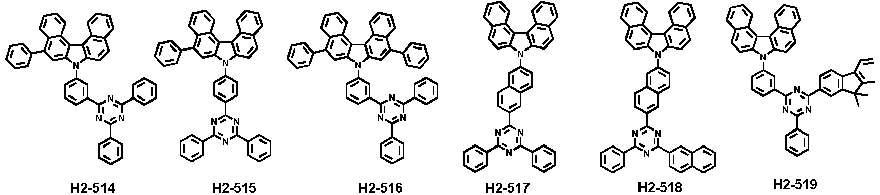

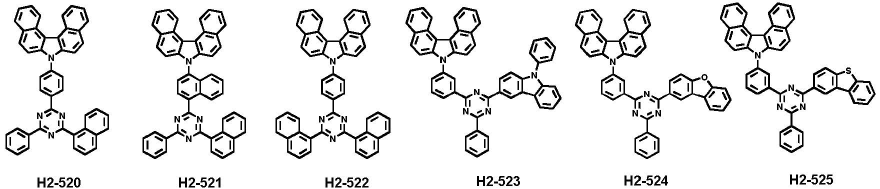

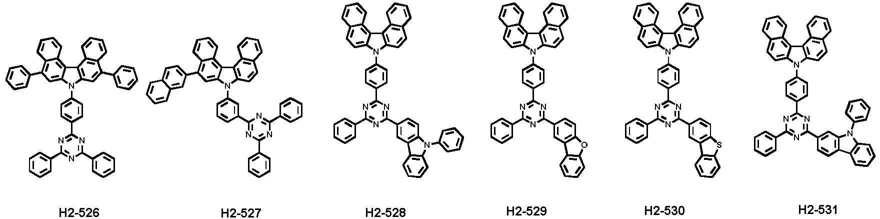

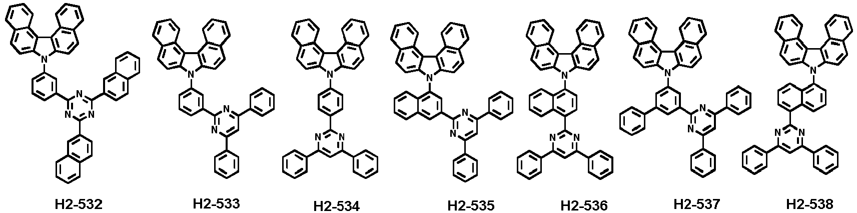

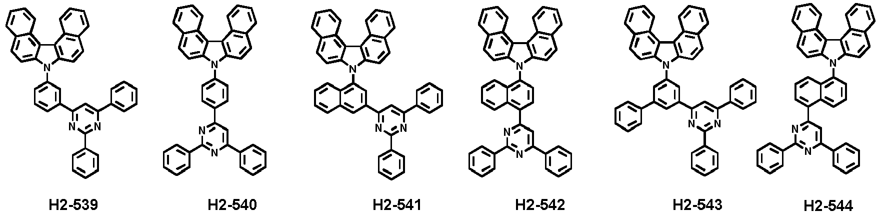

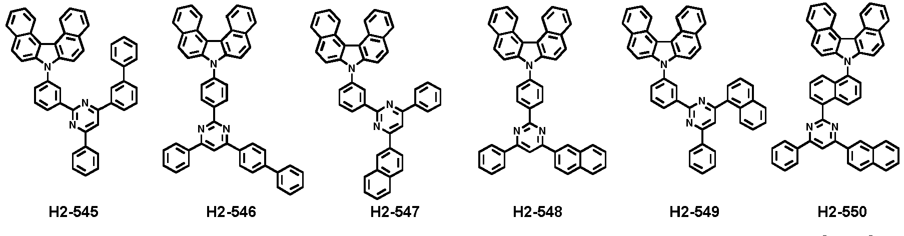

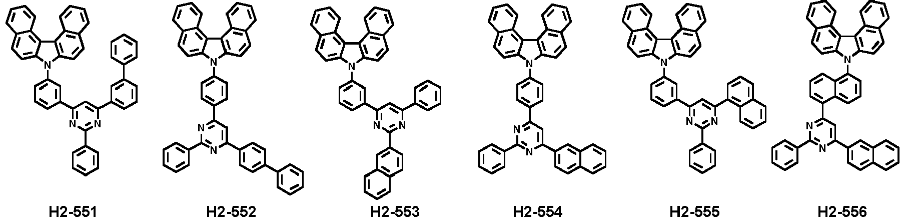

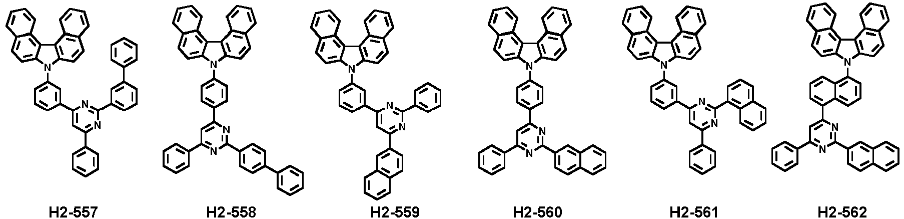

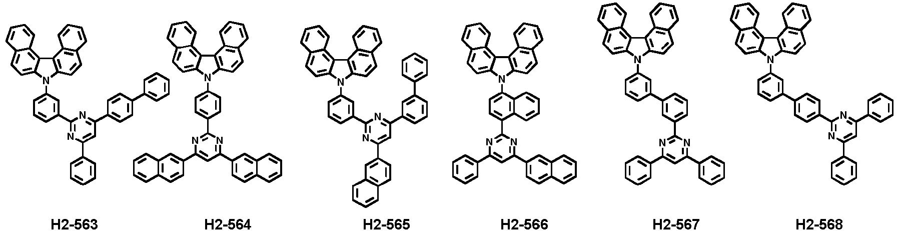

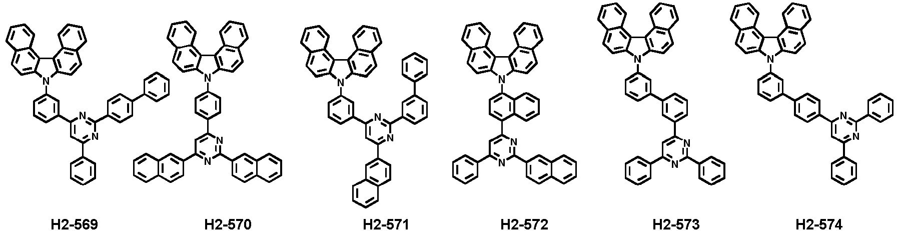

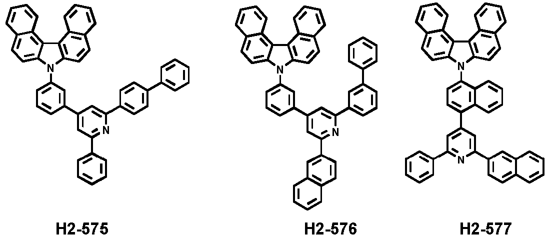

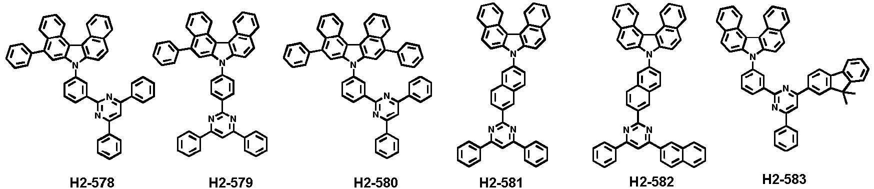

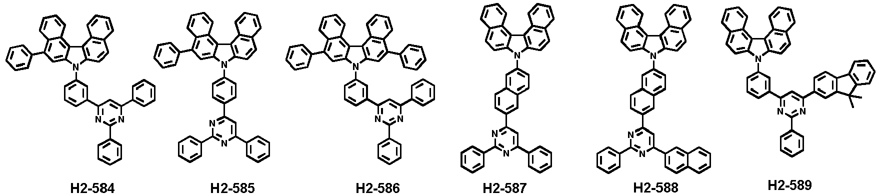

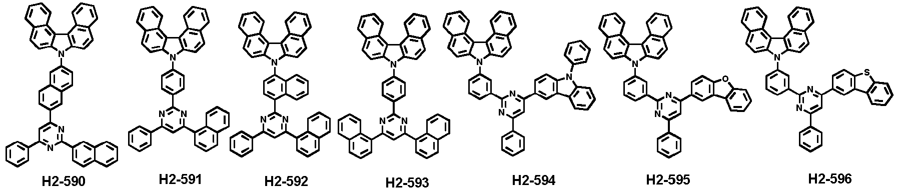

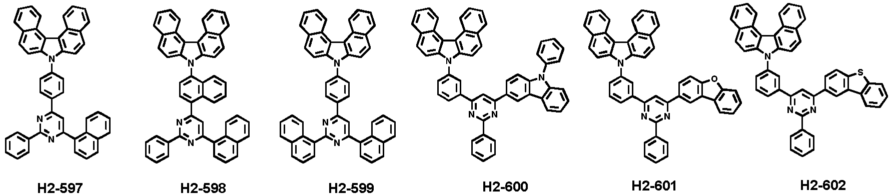

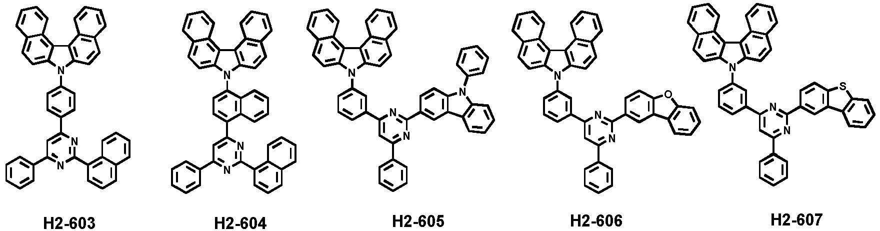

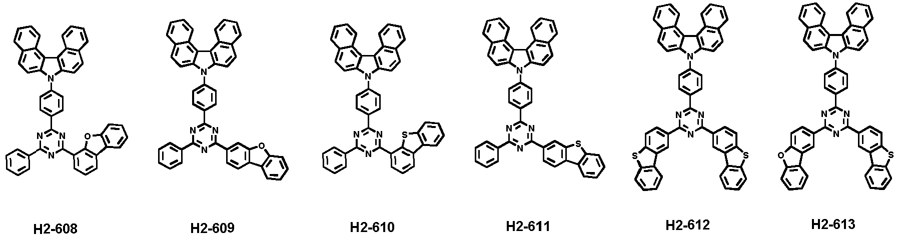

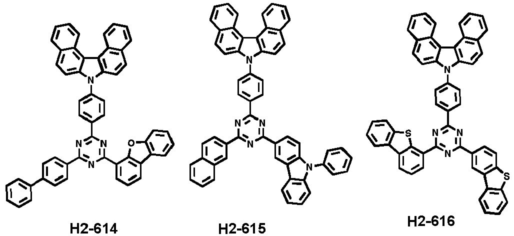

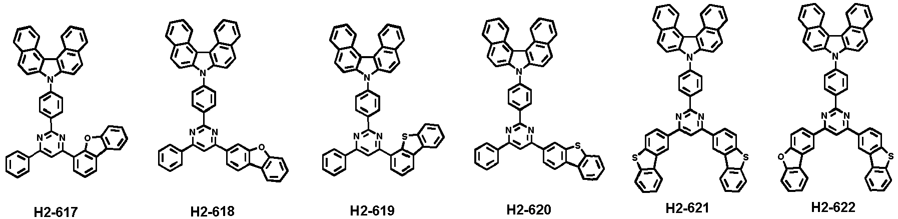

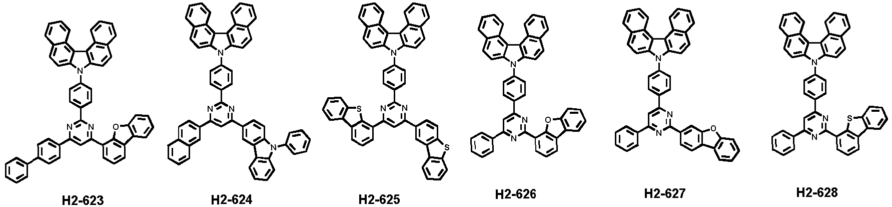

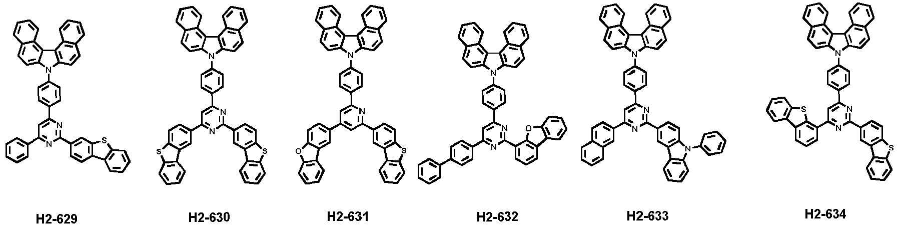

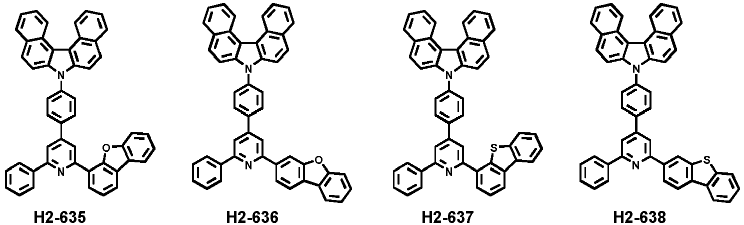

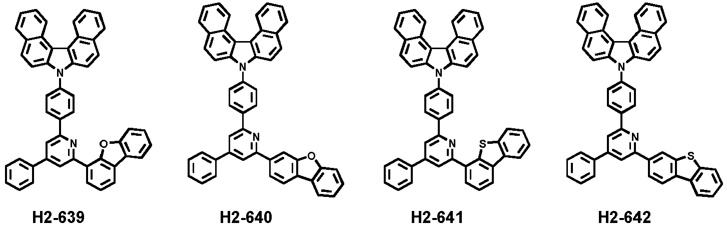

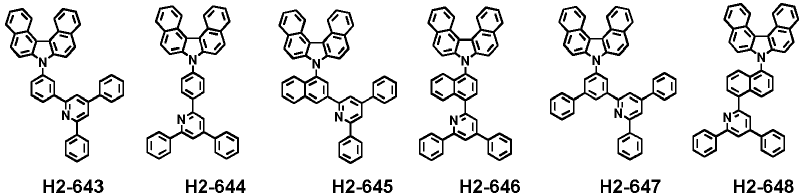

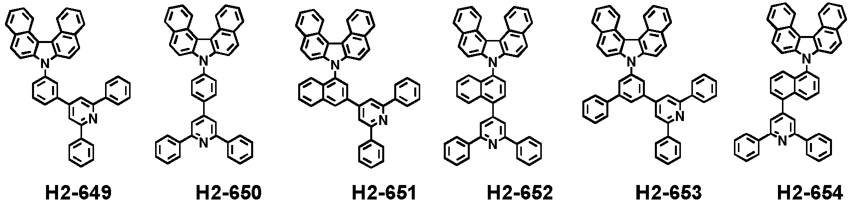

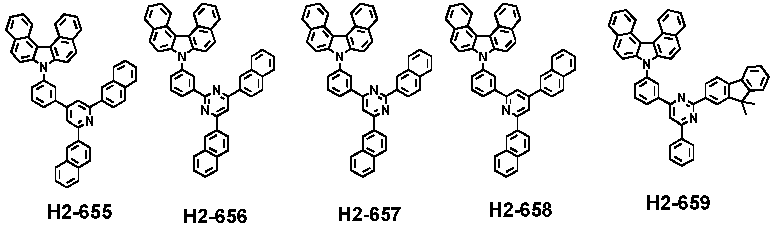

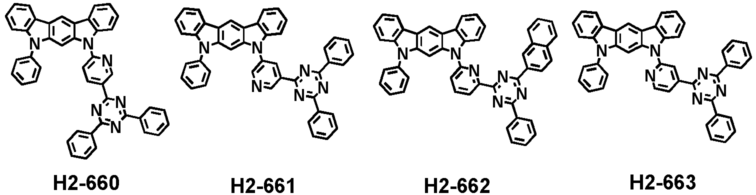

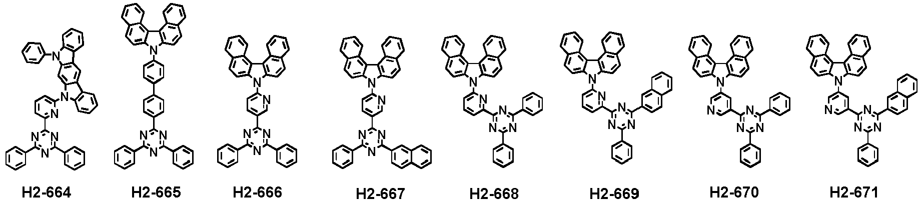

The light-emitting layer means a layer emitting light and may be a single layer or multi-layers consisting of two or more layers. The doping concentration of dopant compounds to host compounds in the light-emitting layer is preferably less than 20 wt%.

The dopants included in the organic EL device of the present invention are preferably one or more phosphorescent dopants. The phosphorescent dopant material applied to the organic electroluminescent device of the present invention is not specifically limited, but preferably may be selected from complex compounds of iridium (Ir), osmium (Os), copper (Cu), and platinum (Pt), more preferably ortho metallated complex compounds of iridium (Ir), osmium (Os), copper (Cu), and platinum (Pt), and even more preferably ortho metallated iridium complex compounds.

The phosphorescent dopants may be selected from the group consisting of the compounds represented by the following formulae 101 to 103: 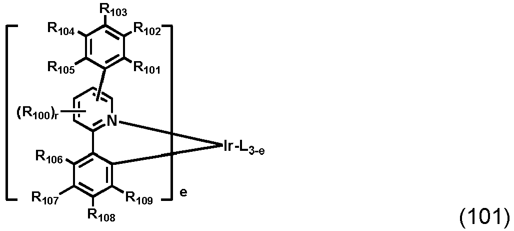 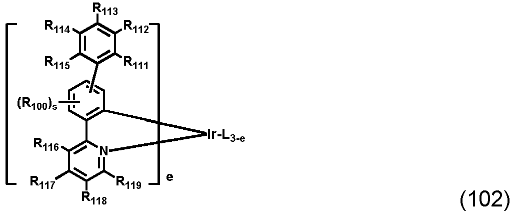 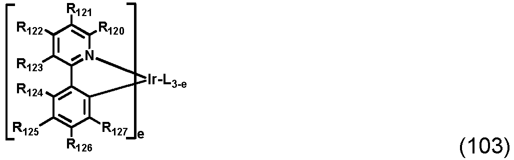 wherein
L is selected from the following structures:

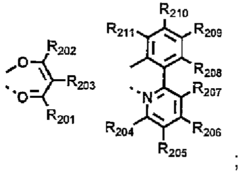

R₁₀₀ represents hydrogen, or a substituted or unsubstituted (C1-C30)alkyl group; R₁₀₁ to R₁₀₉ and R₁₁₁ to R₁₂₃ each independently represent hydrogen, deuterium, a halogen; a (C1-C30)alkyl group unsubstituted or substituted with halogen(s); a cyano group, a substituted or unsubstituted (C1-C30)alkoxy group, a substituted or unsubstituted (C6-C30)aryl group, or a substituted or unsubstituted (C3-C30)cycloalkyl group; R₁₂₀ to R₁₂₃ are linked to an adjacent substituent(s) to form a substituted or unsubstituted mono- or polycyclic, (C3-C30) alicyclic or aromatic ring, for example, quinoline; R₁₂₄ to R₁₂₇ each independently represent hydrogen, deuterium, a halogen, a substituted or unsubstituted (C1-C30)alkyl group, or a substituted or unsubstituted (C6-C30)aryl group; when R₁₂₄ to R₁₂₇ are aryl groups, they are linked to an adjacent substituent(s) to form a substituted or unsubstituted mono- or polycyclic, (C3-C30) alicyclic or aromatic ring, or a heteroaromatic ring, for example, fluorene, dibenzothiophene, or dibenzofuran; R₂₀₁ to R₂₁₁ each independently represent hydrogen, deuterium, a halogen, a (C1-C30)alkyl group unsubstituted or substituted with halogen(s), or a substituted or unsubstituted (C6-C30)aryl group; R₂₀₈ to R₂₁₁ may be linked to an adjacent substituent(s) to form a substituted or unsubstituted mono- or polycyclic, (C3-C30) alicyclic or aromatic ring, or a heteroaromatic ring, for example, fluorene, dibenzothiophene, or dibenzofuran; r and s each independently represent an integer of 1 to 3; where r or s is an integer of 2 or more, each of R₁₀₀ may be the same or different; and e represents an integer of 1 to 3.

The phosphorescent dopant material includes the following:

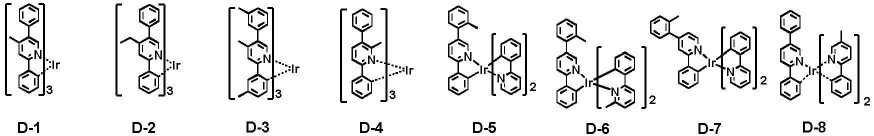

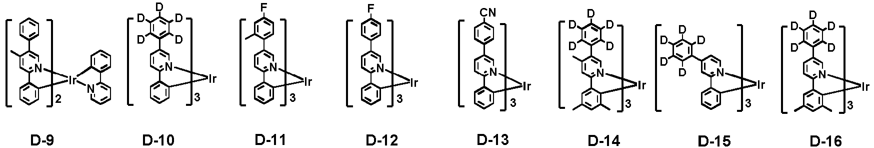

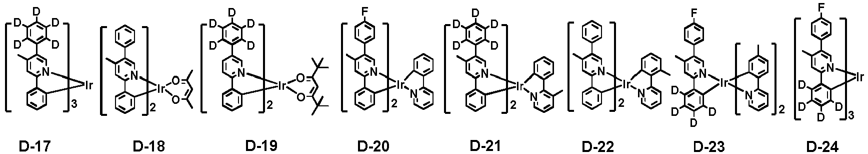

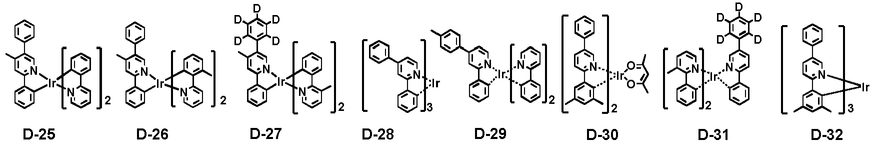

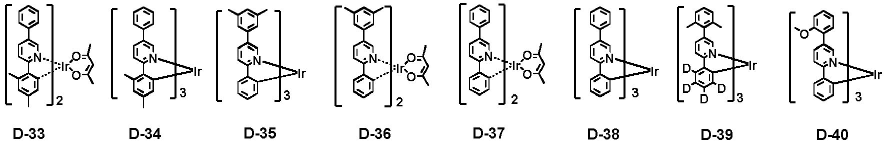

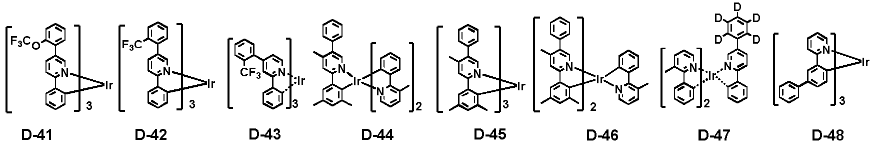

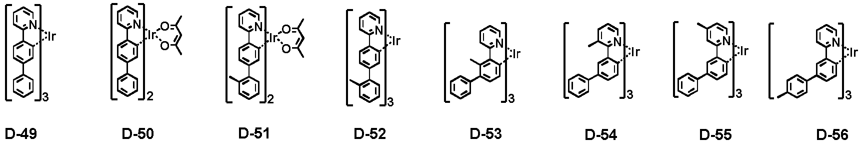

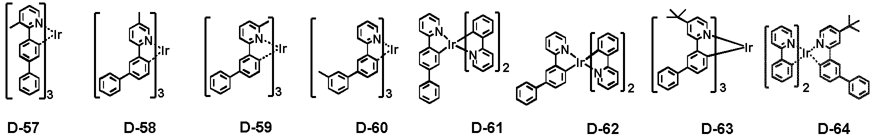

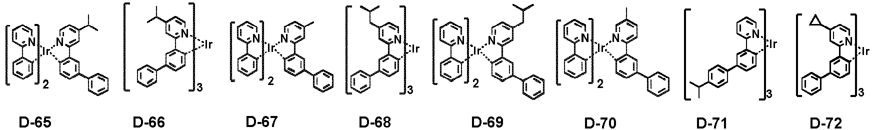

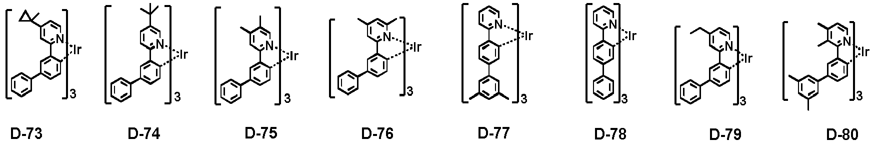

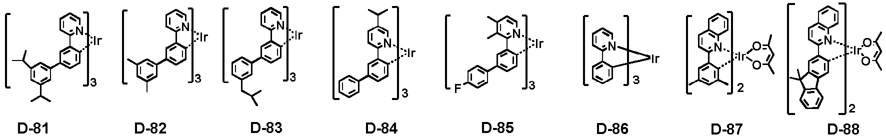

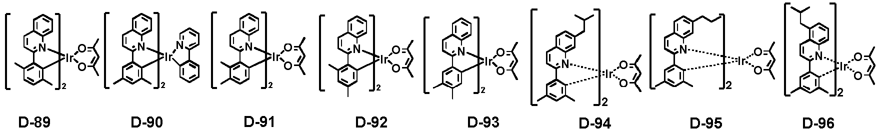

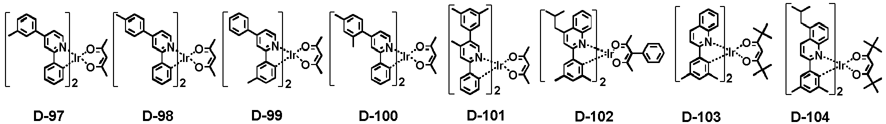

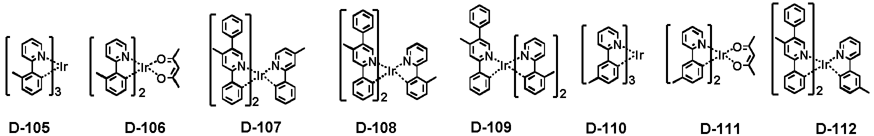

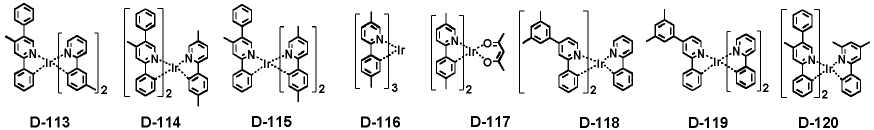

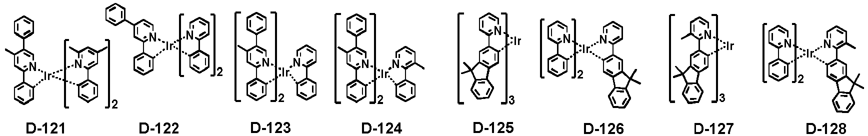

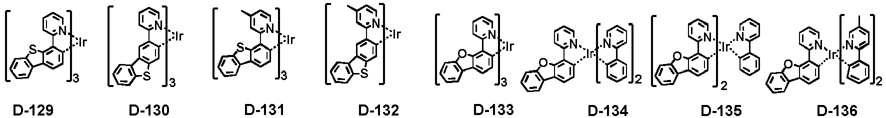

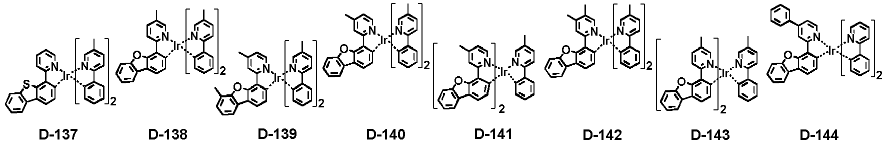

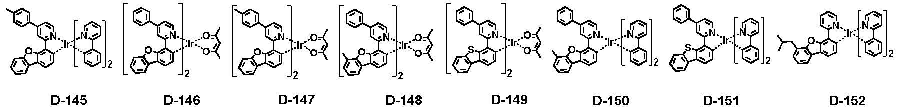

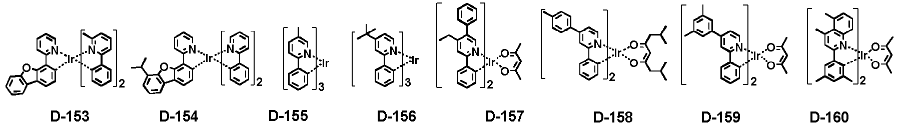

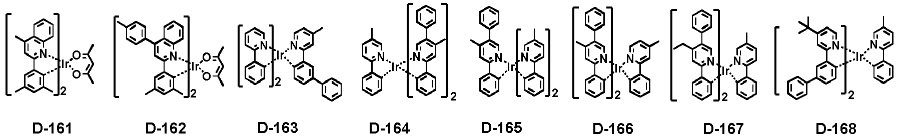

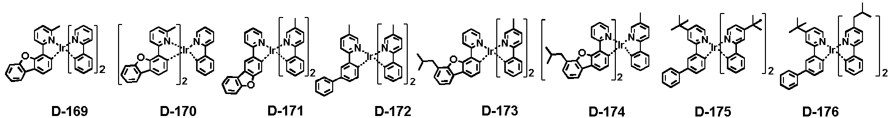

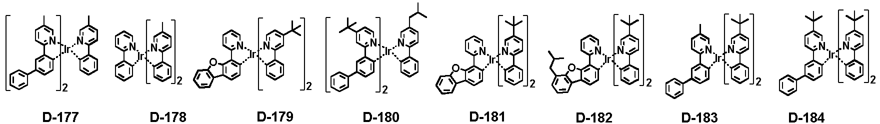

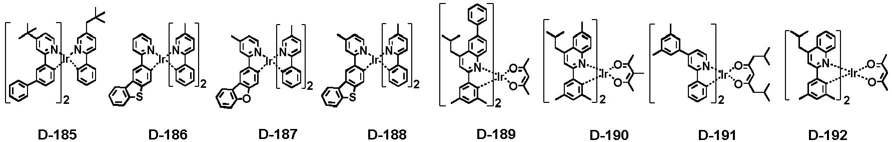

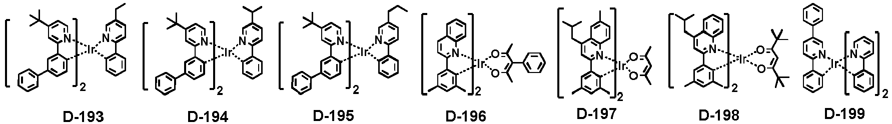

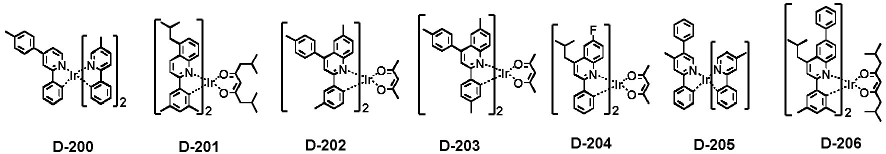

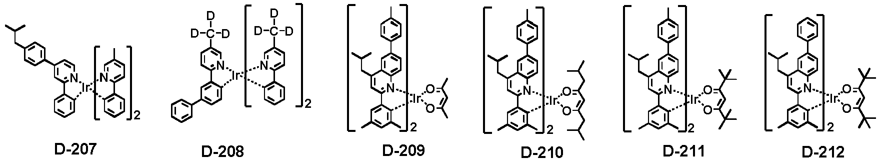

The organic EL device of the present invention may further include at least one compound selected from the group consisting of arylamine-based compounds and styrylarylamine-based compounds in the organic layer.

In the organic EL device of the present invention, the organic layer may further comprise at least one metal selected from the group consisting of metals of Group 1, metals of Group 2, transition metals of the 4^{th} period, transition metals of the 5^{th} period, lanthanides, and organic metals of d-transition elements of the Periodic Table, or at least one complex compound comprising the metal.

Preferably, in the organic electroluminescent device of the present invention, at least one layer (hereinafter, "a surface layer") selected from a chalcogenide layer, a metal halide layer and a metal oxide layer may be placed on an inner surface(s) of one or both electrode(s). Specifically, it is preferred that a chalcogenide (including oxides) layer of silicon or aluminum is placed on an anode surface of a light-emitting medium layer, and a metal halide layer or metal oxide layer is placed on a cathode surface of an electroluminescent medium layer. The surface layer provides operating stability for the organic electroluminescent device. Preferably, the chalcogenide includes SiO_{X}(1≤X≤2), AlO_{X}(1≤X≤1.5), SiON, SiAlON, etc.; the metal halide includes LiF, MgF₂, CaF₂, a rare earth metal fluoride, etc.; and the metal oxide includes Cs₂O, Li₂O, MgO, SrO, BaO, CaO, etc.

A hole injection layer, a hole transport layer, an electron blocking layer, or their combinations can be used between an anode and a light-emitting layer. The hole injection layer may be multi-layers in order to lower a hole injection barrier (or hole injection voltage) from an anode to a hole transport layer or an electron blocking layer, wherein each of the multi-layers simultaneously uses two compounds. The hole transport layer or the electron blocking layer may also be multi-layers.

An electron buffer layer, a hole blocking layer, an electron transport layer, an electron injection layer, or their combinations can be used between a light-emitting layer and a cathode. The electron buffer layer may be multi-layers in order to control the injection of an electron and improve interface properties between the light-emitting layer and the electron injection layer, wherein each of the multi-layers simultaneously uses two compounds. The hole blocking layer or the electron transport layer may also be multi-layers, wherein each of the multi-layers may use a multi-component of compounds.

Preferably, in the organic electroluminescent device of the present invention, a mixed region of an electron transport compound and a reductive dopant, or a mixed region of a hole transport compound and an oxidative dopant may be placed on at least one surface of a pair of electrodes. In this case, the electron transport compound is reduced to an anion, and thus it becomes easier to inject and transport electrons from the mixed region to a light-emitting medium. Further, the hole transport compound is oxidized to a cation, and thus it becomes easier to inject and transport holes from the mixed region to a light-emitting medium. Preferably, the oxidative dopant includes various Lewis acids and acceptor compounds; and the reductive dopant includes alkali metals, alkali metal compounds, alkaline earth metals, rare-earth metals, and mixtures thereof. A reductive dopant layer may be employed as a charge-generating layer to prepare an organic electroluminescent device having two or more light-emitting layers and emitting white light.

In order to form each layer constituting the organic electroluminescent device of the present invention, dry film-forming methods, such as vacuum deposition, sputtering, plasma, ion plating methods, etc., or wet film-forming methods, such as spin coating, dip coating, flow coating methods, etc., can be used. When forming a layer by using a first host and a second host according to the present invention, co-deposition or mixed-deposition may be used.

When using a wet film-forming method, a thin film is formed by dissolving or dispersing the material constituting each layer in suitable solvents, such as ethanol, chloroform, tetrahydrofuran, dioxane, etc. The solvents are not specifically limited as long as the material constituting each layer is soluble or dispersible in the solvents, which do not cause any problems in forming a layer.

Furthermore, a display device or a light device can be produced by using the organic EL device of the present invention.

Hereinafter, the preparation methods of devices by using host compounds and dopant compounds of the present invention will be explained in detail with reference to the following examples:

### Device Example 1-1: not according to the invention:

An OLED device comprising the organic electroluminescent compound of the present invention was produced as follows: A transparent electrode indium tin oxide (ITO) thin film (10 Ω/sq) on a glass substrate for an OLED device (Samsung Coming, Republic of Korea) was subjected to an ultrasonic washing with trichloroethylene, acetone, ethanol, and distilled water, sequentially, and was then stored in isopropanol. Next, the ITO substrate was mounted on a substrate holder of a vacuum vapor depositing apparatus. N⁴,N^{4'}-diphenyl-N⁴,N^{4'}-bis(9-phenyl-9H-carbazole-3-yl)-[1,1'-biphenyl]-4,4'-diamine as HI-1 was introduced into a cell of the vacuum vapor depositing apparatus, and the pressure in the chamber of the apparatus was then controlled to 10⁻⁶ torr. Thereafter, an electric current was applied to the cell to evaporate the introduced material, thereby forming a hole injection layer 1 having a thickness of 80 nm on the ITO substrate. 1,4,5,8,9,12-hexaazatriphenylene hexacarbonitrile as HI-2 was then introduced into another cell of the vacuum vapor depositing apparatus, and an electric current was applied to the cell to evaporate the introduced material, thereby forming a hole injection layer 2 having a thickness of 5 nm on hole injection layer 1. N-([1,1'-biphenyl]-4-yl)-9,9-dimethyl-N-(4-(9-phenyl-9H-carbazole-3-yl)phenyl)-9H-fluorene-2-amine as HT-1 was introduced into one cell of the vacuum vapor depositing apparatus. Thereafter, an electric current was applied to the cell to evaporate the introduced material, thereby forming a hole transport layer 1 having a thickness of 10 nm on hole injection layer 2. N,N-di([1,1'-biphenyl]-4-yl)-4'-(9H-carbazole-9-yl)-[1,1'-biphenyl]-4-amine as HT-2 was then introduced into another cell of the vacuum vapor depositing apparatus, and an electric current was applied to the cell to evaporate the introduced material, thereby forming a hole transport layer 2 having a thickness of 60 nm on hole transport layer 1. Thereafter, compounds H1-1 and H2-2 as hosts were respectively introduced into two cells of the vacuum vapor depositing apparatus and compound D-96 as a dopant was introduced into another cell. The two host materials were evaporated at the same rates of 1:1, and the dopant was evaporated at a different rate and deposited in a doping amount of 3 wt%, based on the total weight of the host and dopant, to form a light-emitting layer having a thickness of 40 nm on the hole transport layer. Next, 2,4-bis(9,9-dimethyl-9H-fluorene-2yl)-6-(naphthalene-2-yl)-1,3,5-triazine as ET-1 and lithium quinolate as EI-1 were evaporated at the same rates of 1:1 on another two cells to form an electron transport layer having a thickness of 30 nm on the light-emitting layer. After depositing lithium quinolate of EI-1 having a thickness of 2 nm as an electron injection layer on the electron transport layer, an Al cathode having a thickness of 80 nm was then deposited by another vacuum vapor deposition apparatus on the electron injection layer. Thus, an OLED device was produced.

The produced OLED device showed the driving voltage at a luminance of 1,000nit, luminescent efficiency, CIE color coordinate, and the lifespan taken to be reduced from 100% to 90% of the constant current at a luminance of 5,000nit as provided in Table 1 below.

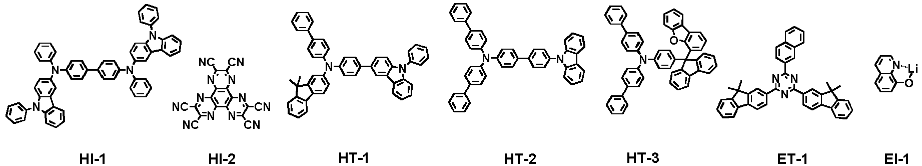

### Comparative Example 1-1:

An OLED device was produced in the same manner as in Device Example 1-1, except that only the second host compound was used as a host in a light-emitting layer.

The luminescent properties of the OLED devices produced in Device Example 1-1 and Comparative Example 1-1 are provided in Table 1 below.

**Table 1**

| | Hole Transport Layer | Host | Dopant | Voltage (V) | Efficiency (cd/A) | Color Coordinate (x, y) | T90 lifespan (hr) |
|---|---|---|---|---|---|---|---|
| Device Example 1-1 | HT-1/HT-2 | H1-1:H2-2 | D-96 | 4.3 | 25.5 | 0.663, 0.336 | 360 |
| Comparative Example 1-1 | HT-1/HT-2 | H2-2 | D-96 | 4.1 | 28.2 | 0.662, 0.337 | 100 |

### Device Examples 2-1 to 2-9 and 2-11 to 2-13 not according to the invention; Device Example 2-10 according to the invention:

An OLED device was produced in the same manner as in Device Example 1-1, except that hole injection layer 2 has a thickness of 3 nm, hole transport layer 1 has a thickness of 40 nm, hole transport layer 2 is not present, D-25 as a dopant was deposited in a doping amount on 15wt% in a light-emitting layer, the electron transport layer having a thickness of 35 nm was deposited via the evaporation rate of 4:6, the combinations of the first host compound and the second host compound used as hosts in a light-emitting layer are based on Device Examples 2-1 to 2-13 as provided in Table 2 below, and the lifespan taken to be reduced from 100% to 90% of the constant current at a luminance of 15,000nit as provided in Table 2 below.

### Device Examples 2-14 to 2-18: not according to the invention:

An OLED device was produced in the same manner as in Device Examples 2-1 to 2-13, except that hole injection layer 2 has a thickness of 3 nm, hole transport layer 1 has a thickness of 40 nm, hole transport layer 2 is not present, D-1 as a dopant was used in a light-emitting layer, the electron transport layer having a thickness of 35 nm was deposited via the evaporation rate of 4:6, the combinations of the first host compound and the second host compound used as hosts in a light-emitting layer are based on Device Examples 2-14 to 2-18 as provided in Table 2 below, and the lifespan taken to be reduced from 100% to 90% of the constant current at a luminance of 15,000nit as provided in Table 2 below.

### Device Examples 3-1 to 3-8: not according to the invention:

An OLED device was produced in the same manner as in Device Examples 2-1 to 2-13, except that hole transport layer 1 has a thickness of 10 nm, hole transport layer 2 of HT-3 has a thickness of 30 nm, D-136 as a dopant was used in a light-emitting layer, and the combinations of the first host compound and the second host compound used as hosts in a light-emitting layer are based on Device Examples 3-1 to 3-8 as provided in Table 2 below.

### Device Example 3-9: not according to the invention:

An OLED device was produced in the same manner as in Device Examples 2-1 to 2-13, except that hole transport layer 1 has a thickness of 10 nm, hole transport layer 2 of HT-3 has a thickness of 30 nm, D-164 as a dopant was used in a light-emitting layer, and the combinations of the first host compound and the second host compound used as hosts in a light-emitting layer are based on Device Example 3-9 as provided in Table 2 below.

### Device Examples 3-10 and 3-11 not according to the invention: Device Example 3-12 according to the invention:

An OLED device was produced in the same manner as in Device Examples 2-1 to 2-13, except that hole transport layer 1 has a thickness of 10 nm, hole transport layer 2 of HT-3 has a thickness of 30 nm, D-168 as a dopant was used in a light-emitting layer, and the combinations of the first host compound and the second host compound used as hosts in a light-emitting layer are based on Device Examples 3-10 to 3-12 as provided in Table 2 below.

### Device Example 3-13: according to the invention:

An OLED device was produced in the same manner as in Device Examples 2-1 to 2-13, except that hole transport layer 1 has a thickness of 10 nm, hole transport layer 2 of HT-3 has a thickness of 30 nm, D-180 as a dopant was used in a light-emitting layer, and the combinations of the first host compound and the second host compound used as hosts in a light-emitting layer are based on Device Example 3-13 as provided in Table 2 below.

### Comparative Examples 2-1 to 2-3:

An OLED device was produced in the same manner as in Device Examples 2-1 to 2-13, except that the first host compound used as hosts in a light-emitting layer is based on Comparative Examples 2-1 to 2-3 as provided in Table 2 below.

### Comparative Examples 3-1 to 3-9:

An OLED device was produced in the same manner as in Device Examples 2-1 to 2-13, except that the second host compound used as hosts in a light-emitting layer is based on Comparative Examples 3-1 to 3-9 as provided in Table 2 below.

### Comparative Example 4-1:

An OLED device was produced in the same manner as in Device Examples 3-1 to 3-8, except that the second host compound used as hosts in a light-emitting layer is based on Comparative Example 4-1 as provided in Table 2 below.

The luminescent properties of the OLED devices produced in the above Device Examples and Comparative Examples are provided in Table 2 below.

**Table 2**

| Only Device Examples 2-10, 3-12 and 3-13 according to the invention, all of the remaining examples in the following table are not within the scope of the invention. | | | | | | | |
|---|---|---|---|---|---|---|---|
| | Hole Transport Layer | Host | Dopant | Voltage [V] | Efficiency [cd/A] | Color Coordinate (x, y) | T90 lifespan [hr] |
| Device Example 2-1 | HT-1 | H1-1:H2-25 | D-25 | 3.3 | 43.2 | 0.297, 0.660 | 100 |
| Device Example 2-2 | HT-1 | H1-1:H2-31 | D-25 | 3 | 58.8 | 0.303, 0.657 | 143 |
| Device Example 2-3 | HT-1 | H1-1:H2-48 | D-25 | 2.8 | 55.3 | 0.302, 0.657 | 124 |
| Device Example 2-4 | HT-1 | H1-1:H2-34 | D-25 | 3 | 55.7 | 0.302, 0.657 | 127 |
| Device Example 2-5 | HT-1 | H1-11:H2-31 | D-25 | 2.9 | 56.9 | 0.306, 0.656 | 147 |
| Device Example 2-6 | HT-1 | H1-12:H2-31 | D-25 | 2.9 | 54.5 | 0.304, 0.657 | 206 |
| Device Example 2-7 | HT-1 | H1-14:H2-31 | D-25 | 3.1 | 49.1 | 0.306, 0.655 | 124 |
| Device Example 2-8 | HT-1 | H1-4:H2-31 | D-25 | 2.9 | 55.2 | 0.300, 0.657 | 131 |
| Device Example 2-9 | HT-1 | H1-35:H2-31 | D-25 | 2.9 | 55.6 | 0.303, 0.656 | 161 |
| Device Example 2-10 | HT-1 | H1-1:H2-101 | D-25 | 3 | 55.6 | 0.303, 0.656 | 124 |
| Device Example 2-11 | HT-1 | H1-9:H2-31 | D-25 | 2.9 | 56 | 0.301, 0.657 | 203 |
| Device Example 2-12 | HT-1 | H1-2:H2-31 | D-25 | 2.8 | 54.9 | 0.307, 0.656 | 116 |
| Device Example 2-13 | HT-1 | H1-34:H2-31 | D-25 | 3 | 52.5 | 0.303, 0.657 | 160 |
| Device Example 2-14 | HT-1 | H1-1:H2-31 | D-1 | 2.8 | 57.8 | 0.315, 0.658 | 254 |
| Device Example 2-15 | HT-1 | H1-1:H2-48 | D-1 | 2.8 | 60.2 | 0.316, 0.659 | 240 |
| Device Example 2-16 | HT-1 | H1-11:H2-31 | D-1 | 2.8 | 52.4 | 0.317, 0.658 | 274 |
| Device Example 2-17 | HT-1 | H1-11:H2-48 | D-1 | 2.7 | 54.3 | 0.316, 0.659 | 272 |
| Device Example 2-18 | HT-1 | H1-11:H2-87 | D-1 | 2.9 | 51.9 | 0.319, 0.655 | 240 |
| Device | HT-1/HT-3 | H1-1:H2-30 | D-136 | 3.3 | 63.9 | 0.324, 0.660 | 240 |
| Example 3-1 | | | | | | | |
| Device Example 3-2 | HT-1/HT-3 | H1-1:H2-31 | D-136 | 3.2 | 71.2 | 0.326, 0.659 | 265 |
| Device Example 3-3 | HT-1/HT-3 | H1-1:H2-48 | D-136 | 3.1 | 68 | 0.325, 0.659 | 265 |
| Device Example 3-4 | HT-1/HT-3 | H1-1:H2-87 | D-136 | 3.3 | 67.4 | 0.327, 0.658 | 290 |
| Device Example 3-5 | HT-1/HT-3 | H1-11:H2-31 | D-136 | 3.1 | 69.2 | 0.327, 0.658 | 292 |
| Device Example 3-6 | HT-1/HT-3 | H1-11:H2-48 | D-136 | 3.2 | 64 | 0.326, 0.658 | 322 |
| Device Example 3-7 | HT-1/HT-3 | H1-11: H2-87 | D-136 | 3.1 | 65.2 | 0.327, 0.657 | 367 |
| Device Example 3-8 | HT-1/HT-3 | H1-35:H2-125 | D-136 | 3.1 | 65.2 | 0.330, 0.655 | 408 |
| Device Example 3-9 | HT-1/HT-3 | H1-35:H2-31 | D-164 | 3.2 | 61.5 | 0.316, 0.656 | 241 |
| Device Example 3-10 | HT-1/HT-3 | H1-1:H2-31 | D-168 | 3.2 | 62.1 | 0.281, 0.665 | 148 |
| Device Example 3-11 | HT-1/HT-3 | H1-35:H2-31 | D-168 | 3.2 | 59.4 | 0.278, 0.668 | 162 |
| Device Example 3-12 | HT-1/HT-3 | H1-12:H2-125 | D-168 | 3.1 | 56.6 | 0.288, 0.665 | 164 |
| Device Example 3-13 | HT-1/HT-3 | H1-12:H2-125 | D-180 | 3.1 | 49.7 | 0.291, 0.664 | 240 |
| Comparative Example 2-1 | HT-1 | H1-12 | D-25 | 5.9 | 3.1 | 0.299, 0.656 | × |
| Comparative Example 2-2 | HT-1 | H1-4 | D-25 | 6.7 | 3 | 0.289, 0.658 | × |
| Comparative Example 2-3 | HT-1 | H1-35 | D-25 | 6.6 | 3.9 | 0.395, 0.658 | × |
| Comparative Example 3-1 | HT-1 | H2-25 | D-25 | 3.1 | 54.2 | 0.308, 0.655 | 45 |
| Comparative Example 3-2 | HT-1 | H2-31 | D-25 | 2.9 | 42.8 | 0.314, 0.652 | 39 |
| Comparative Example 3-3 | HT-1 | H2-48 | D-25 | 2.6 | 49.6 | 0.314, 0.652 | 67 |
| Comparative Example 3-4 | HT-1 | H2-101 | D-25 | 2.8 | 50.3 | 0.315, 0.651 | 24 |
| Comparative Example 3-5 | HT-1 | H2-34 | D-25 | 2.7 | 49.2 | 0.312, 0.652 | 38 |
| Comparative Example 3-6 | HT-1 | H2-30 | D-25 | 2.8 | 55.3 | 0.314, 0.652 | 70 |
| Comparative Example 3-7 | HT-1 | H2-31 | D-1 | 2.9 | 33.5 | 0.323, 0.653 | 130 |
| Comparative Example 3-8 | HT-1 | H2-48 | D-1 | 2.6 | 41.2 | 0.325, 0.653 | 124 |
| Comparative Example 3-9 | HT-1 | H2-87 | D-1 | 2.8 | 37.9 | 0.323, 0.653 | 146 |
| Comparative Example 4-1 | HT-1/HT-3 | H2-125 | D-136 | 3.0 | 64.9 | 0.337, 0.649 | 124 |

### Device Examples 4-1 to 4-7: Examples 4-1, 4-5 and 4-6 are not according to the invention:

An OLED device was produced in the same manner as in Device Example 1-1, except that HT-4 was used as a hole transport layer 2, the combinations of the first host compound and the second host compound used as hosts in a light-emitting layer are based on Device Examples 4-1 to 4-7 as provided in Table 3 below, and the lifespan taken to be reduced from 100% to 95% of the constant current at a luminance of 5,000nit as provided in Table 3 below.

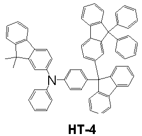

### Comparative Example 5-1 and 5-2:

An OLED device was produced in the same manner as in Device Examples 4-1 to 4-7, except that the second host compound used as hosts in a light-emitting layer is based on Comparative Examples 5-1 and 5-2 as provided in Table 3 below.

The luminescent properties of the OLED devices produced in Device Examples 4-1 to 4-7, and Comparative Examples 5-1 and 5-2 are provided in Table 3 below. Examples 4-1, 4-5 and 4-6 are not according to the invention:

**Table 3**

| | Hole Transport Layer | Host | Dopant | Voltage [V] | Efficiency [cd/A] | Color Coordinate (x, y) | T95 lifespan [hr] |
|---|---|---|---|---|---|---|---|
| Device Example 4-1 | HT-1 / HT-4 | H1-287 : H2-496 | D-96 | 3.8 | 30.8 | 0.667, 0.333 | 310 |
| Device Example 4-2 | HT-1 / HT-4 | H1-12 : H2-504 | D-96 | 3.5 | 30.7 | 0.667, 0.333 | 390 |
| Device Example 4-3 | HT-1 / HT-4 | H1-9 : H2-496 | D-96 | 3.9 | 31.1 | 0.665, 0.335 | 130 |
| Device Example 4-4 | HT-1 / HT-4 | H1-35 : H2-496 | D-96 | 3.8 | 31.1 | 0.665, 0.334 | 200 |
| Device Example 4-5 | HT-1 / HT-4 | H1-287 : H2-504 | D-96 | 3.7 | 31.3 | 0.666, 0.333 | 200 |
| Device Example 4-6 | HT-1 / HT-4 | H1-282 : H2-504 | D-96 | 3.7 | 31.4 | 0.666, 0.334 | 120 |
| Device Example 4-7 | HT-1 / HT-4 | H1-12 : H2-496 | D-96 | 3.6 | 29.2 | 0.667, 0.333 | 150 |
| Comparative Example 5-1 | HT-1 / HT-4 | H2-496 | D-96 | 3.7 | 31.0 | 0.665, 0.334 | 90 |
| Comparative Example 5-2 | HT-1 / HT-4 | H2-504 | D-96 | 3.7 | 31 | 0.667, 0.333 | 70 |

### Device Examples 5-1 to 5-2: not according to the invention:

An OLED device was produced in the same manner as in Device Examples 3-1 to 3-11, except that D-134 was used as a dopant in a light-emitting layer, the combinations of the first host compound and the second host compound used as hosts in a light-emitting layer are based on Device Examples 5-1 and 5-2 as provided in Table 4 below, and the lifespan taken to be reduced from 100% to 97% of the constant current at a luminance of 15,000nit as provided in Table 4 below.

### Comparative Examples 6-1 and 6-2:

An OLED device was produced in the same manner as in Device Examples 5-1 and 5-2, except that the first host compound used as hosts in a light-emitting layer is based on Comparative Examples 6-1 and 6-2 as provided in Table 4 below.

### Comparative Example 7-1:

An OLED device was produced in the same manner as in Device Examples 5-1 and 5-2, except that the second host compound used as hosts in a light-emitting layer is based on Comparative Example 7-1 as provided in Table 4 below.

The luminescent properties of the OLED devices produced in Device Examples 5-1 and 5-2, Comparative Examples 6-1 and 6-2, and Comparative Example 7-1 are provided in Table 4 below.

**Table 4**

| | Hole Transport Layer | Host | Dopant | Voltage [V] | Efficiency [cd/A] | Color Coordinate (x, y) | T97 lifespan [hr] |
|---|---|---|---|---|---|---|---|
| Device Example 5-1 | HT-1/ HT-3 | H1-12 : H2-660 | D-134 | 3.1 | 63.2 | 0.313, 0.665 | 39 |
| Device Example 5-2 | HT-1/ HT-3 | H1-35 : H2-660 | D-134 | 3.2 | 64.8 | 0.312, 0.665 | 56 |
| Comparative Example 6-1 | HT-1/ HT-3 | H1-12 | D-134 | 6.4 | 2.9 | 0.305, 0.660 | × |
| Comparative Example | HT-1/ HT-3 | H1-35 | D-134 | 7.2 | 3.5 | 0.302, 0.664 | × |
| 6-2 | | | | | | | |
| Comparative Example 7-1 | HT-1/ HT-3 | H2-660 | D-134 | 3.0 | 55.4 | 0.321, 0.659 | 5 |

The organic electroluminescent device of the present invention provides longer lifespan compared with conventional devices.

## Claims

1. An organic electroluminescent device comprising at least one light-emitting layer between an anode and a cathode, wherein the light-emitting layer comprises a host and a phosphorescent dopant; the host consists of multi-component host compounds; at least a first host compound of the multi-component host compounds is represented by the following formula 1 which is a bicarbazole derivative containing an aryl group, and a second host compound is represented by the following formula 2 which is a carbazole derivative including a nitrogen-containing heteroaryl group: 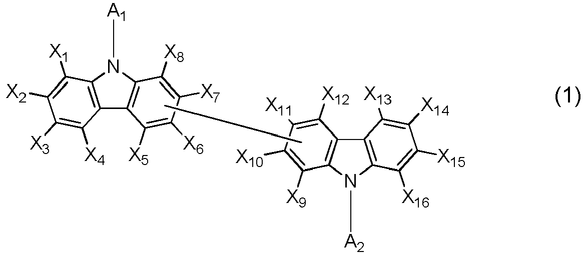 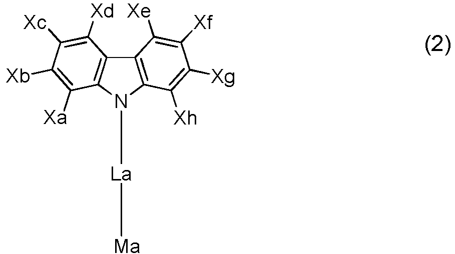 wherein
A₁ represents unsubstituted phenyl, unsubstituted biphenyl;
A₂ represents unsubstituted phenyl or unsubstituted biphenyl;
X₁ to X₁₆ each independently represent hydrogen, deuterium, an unsubstituted (C6-C60) aryl group or an unsubstituted 3- to 30-membered heteroaryl group;
Ma represents substituted or unsubstituted triazinyl;
La represents a substituted or unsubstituted (C6-C30)arylene group; and, wherein the compound of formula 2 is represented by the following formula 7 or 9: 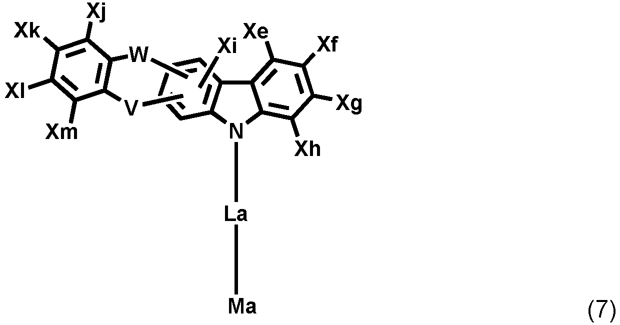 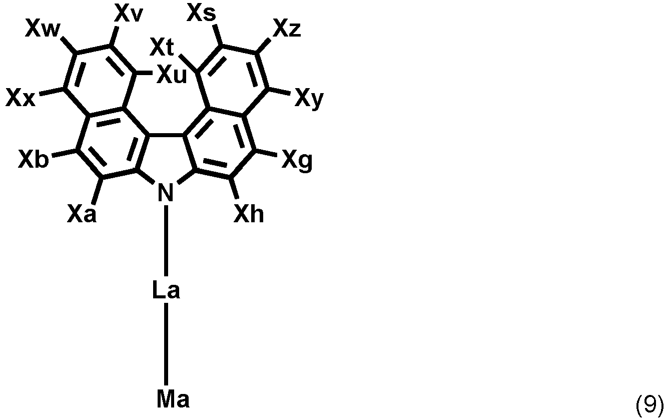 wherein
V represents a single bond, CR₁₆R₁₇, S or O; and W represents CR₁₆R₁₇, S or O;
Xa, Xb, Xj to Xm, Xe to Xh, and Xs to Xz each independently represent hydrogen, deuterium, a substituted or unsubstituted (C6-C60) aryl group
Xi represents hydrogen, deuterium, a halogen, a cyano group, a substituted or unsubstituted
(C1-C30)alkyl group, a substituted or unsubstituted (C2-C30)alkenyl group, a
substituted or unsubstituted (C2-C30)alkynyl group, a substituted or unsubstituted
(C3-C30)cycloalkyl group, a substituted or unsubstituted (C6-C60)aryl group, a substituted
or unsubstituted 3- to 30-membered heteroaryl group, a substituted or unsubstituted
tri(C1-C30)alkylsilyl group, a substituted or unsubstituted tri(C6-C30)arylsilyl
group, a substituted or unsubstituted di(C1-C30)alkyl(C6-C30)arylsilyl group, or a substituted or unsubstituted mono- or di-(C6-C30)arylamino group; or are linked
between adjacent substituents to form a substituted or unsubstituted mono- or
polycyclic, (C3-C30) alicyclic or aromatic ring whose carbon atom(s) ring may be
replaced with at least one hetero atom selected from nitrogen, oxygen and sulfur;
R₅ to R₉ each independently represent a substituted or unsubstituted (C1-C30)alkyl group, a substituted or unsubstituted (C6-C60)aryl group, or a substituted or unsubstituted 3- to 30-membered heteroaryl group; and
R₁₆ and R₁₇ each independently represent a substituted or unsubstituted (C1-C30)alkyl group, a substituted or unsubstituted (C3-C30)cycloalkyl group, a substituted or unsubstituted (C6-C60)aryl group, or a substituted or unsubstituted 3-to 30-membered heteroaryl group.

## Patentansprüche

1. Organische Elektrolumineszenzvorrichtung, umfassend mindestens eine lichtemittierende Schicht zwischen einer Anode und einer Kathode, wobei die lichtemittierende Schicht einen Wirt und ein phosphoreszierendes Dotiermittel umfasst; der Wirt aus Multikomponentenwirtsverbindungen besteht; mindestens eine erste Wirtsverbindung der Multikomponentenwirtsverbindungen durch die folgende Formel 1 dargestellt ist, die ein Bicarbazolderivat ist, das eine Arylgruppe enthält, und eine zweite Wirtsverbindung durch die folgende Formel 2 dargestellt ist, die ein Carbazolderivat ist, das eine stickstoffhaltige Heteroarylgruppe einschließt: 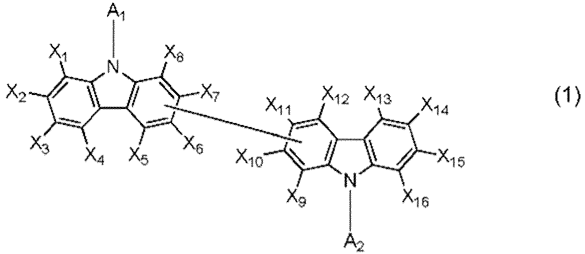 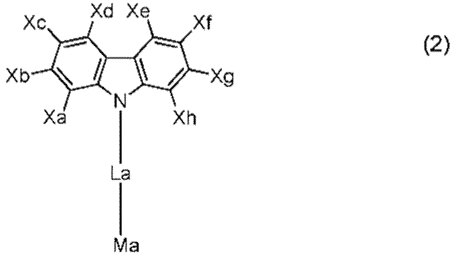 wobei
A₁ unsubstituiertes Phenyl, unsubstituiertes Biphenyl darstellt;
A₂ unsubstituiertes Phenyl oder unsubstituiertes Biphenyl darstellt;
X₁ bis X₁₆ jeweils unabhängig Wasserstoff, Deuterium, eine unsubstituierte (C6-C60)-Arylgruppe oder eine unsubstituierte 3- bis 30-gliedrige Heteroarylgruppe darstellen;
Ma substituiertes oder unsubstituiertes Triazinyl darstellt;
La eine substituierte oder unsubstituierte (C6-C30)-Arylengruppe darstellt; und
wobei die Verbindung der Formel 2 durch die folgende Formel 7 oder 9 dargestellt ist: 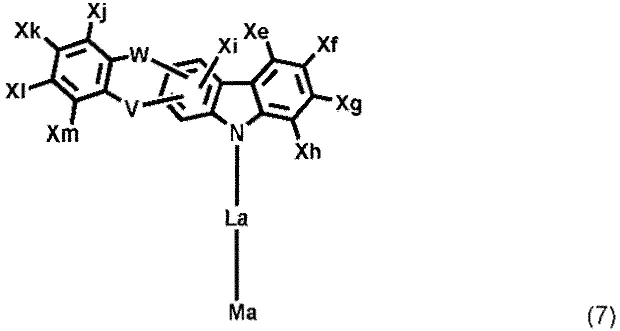 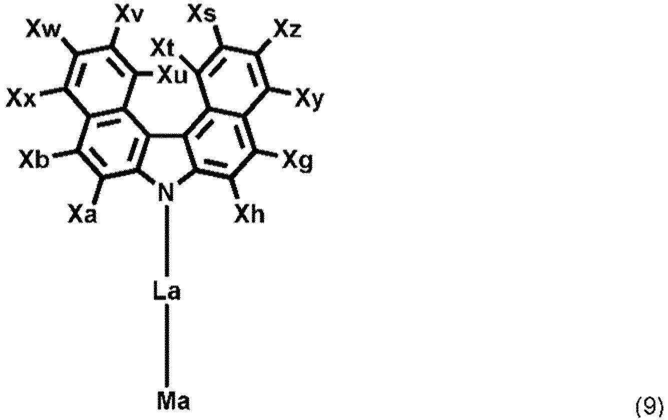 wobei
V eine Einzelbindung, CR₁₆R₁₇, S oder O darstellt; und W CR₁₆R₁₇, S oder O darstellt;
Xa, Xb, Xj bis Xm, Xe bis Xh, und Xs bis Xz jeweils unabhängig Wasserstoff, Deuterium, eine substituierte oder unsubstituierte (C6-C60)-Arylgruppe darstellen;
Xi Wasserstoff, Deuterium, ein Halogen, eine Cyanogruppe, eine substituierte oder unsubstituierte (C1-C30)-Alkylgruppe, eine substituierte oder unsubstituierte (C2-C30)-Alkenylgruppe, eine substituierte oder unsubstituierte (C2-C30)-Alkynylgruppe, eine substituierte oder unsubstituierte (C3-C30)-Cycloalkylgruppe, eine substituierte oder unsubstituierte (C6-C60)-Arylgruppe, eine substituierte oder unsubstituierte 3- bis 30-gliedrige Heteroarylgruppe, eine substituierte oder unsubstituierte Tri(C1-C30)-Alkylsilylgruppe, eine substituierte oder unsubstituierte Tri(C6-C30)-arylsilylgruppe, eine substituierte oder unsubstituierte Di(C1-C30)-alkyl(C6-C30)-arylsilylgruppe oder eine substituierte oder unsubstituierte Mono- oder Di-(C6-C30)-arylaminogruppe darstellt; oder zwischen anliegenden Substituenten verknüpft sind, um einen substituierten oder unsubstituierten mono- oder polycyclischen, alicyclischen oder aromatischen (C3-C30)-Ring zu bilden, dessen Kohlenstoffatom(e) durch mindestens ein Heteroatom ersetzt werden kann/können, das unter Stickstoff, Sauerstoff und Schwefel ausgewählt ist;
R₅ bis R₉ jeweils unabhängig eine substituierte oder unsubstituierte (C1-C30)-Alkylgruppe, eine substituierte oder unsubstituierte (C6-C60)-Arylgruppe, oder eine substituierte oder unsubstituierte 3- bis 30-gliedrige Heteroarylgruppe darstellen; und
R₁₆ und R₁₇ jeweils unabhängig eine substituierte oder unsubstituierte (C1-C30)-Alkylgruppe, eine substituierte oder unsubstituierte (C3-C30)-Cycloalkylgruppe, eine substituierte oder unsubstituierte (C6-C60)-Arylgruppe, oder eine substituierte oder unsubstituierte 3- bis 30-gliedrige Heteroarylgruppe darstellen.

## Revendications

1. Dispositif organique électroluminescent comprenant au moins une couche émettant de la lumière entre une anode et une cathode, dans lequel la couche émettant de la lumière comprend un hôte et un dopant phosphorescent ; l'hôte est constitué de composés hôtes à composants multiples ; au moins un premier composé hôte des composés hôtes à composants multiples est représenté par la formule suivante 1 qui est un dérivé du bicarbazole contenant un groupe aryle, et un deuxième composé hôte est représenté par la formule suivante 2 qui est un dérivé du carbazole incluant un groupe hétéroaryle contenant de l'azote : 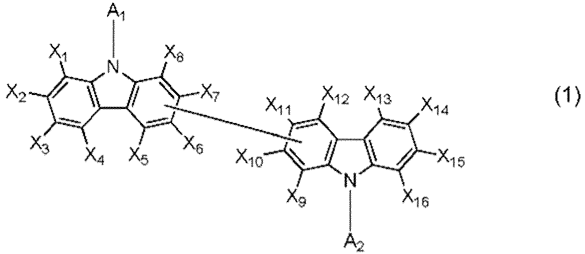 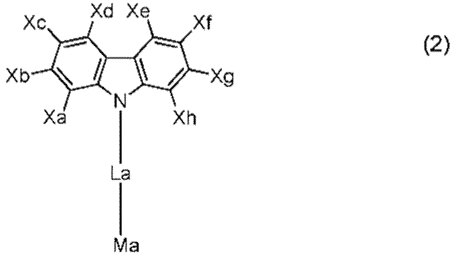 où
A₁ représente un groupe phényle non substitué, biphényle non substitué ;
A₂ représente un groupe phényle non substitué ou biphényle non substitué ;
X₁ à X₁₆ représentent chacun indépendamment un hydrogène, un deutérium, un groupe aryle en (C₆ à C₆₀) non substitué ou un groupe hétéroaryle à 3 à 30 chaînons non substitué ;
Ma représente un groupe triazinyle substitué ou non substitué ;
La représente un groupe arylène en (C₆ à C₃₀) substitué ou non substitué ; et,
dans lequel le composé de formule 2 est représenté par la formule suivante 7 ou 9 : 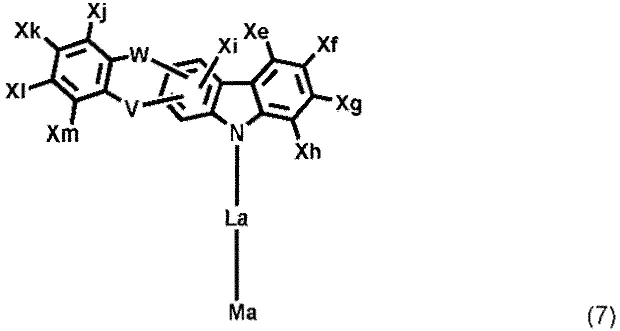 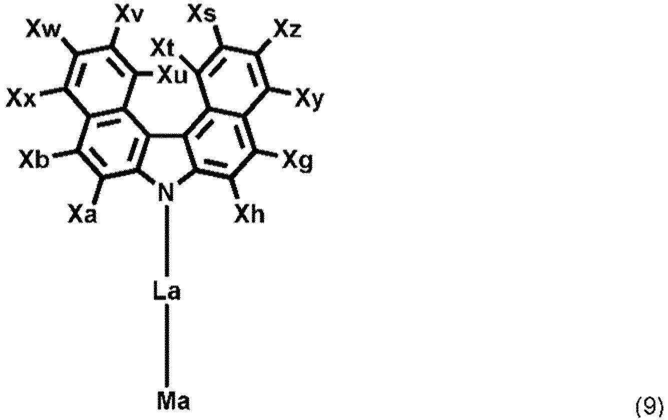 où
V représente une liaison simple, CR₁₆R₁₇, S ou O ; et W représente CR₁₆R₁₇, S ou O ;
Xa, Xb, Xj à Xm, Xe à Xh, et Xs à Xz représentent chacun indépendamment un hydrogène, un deutérium, un groupe aryle en (C₆ à C₆₀) substitué ou non substitué
Xi représente un hydrogène, un deutérium, un groupe halogéno, un groupe cyano, un groupe alkyle en (C₁ à C₃₀) substitué ou non substitué, un groupe alcényle en (C₂ à C₃₀) substitué ou non substitué, un groupe alcynyle en (C₂ à C₃₀) substitué ou non substitué, un groupe cycloalkyle en (C₃ à C₃₀) substitué ou non substitué, un groupe aryle en (C₆ à C₆₀) substitué ou non substitué, un groupe hétéroaryle à 3 à 30 chaînons substitué ou non substitué, un groupe tri(alkylsilyle en C₁ à C₃₀) substitué ou non substitué, un groupe tri(arylsilyle en C₆ à C₃₀) substitué ou non substitué, un groupe di(alkyle en C₁ à C₃₀)(arylsilyle en C₆ à C₃₀) substitué ou non substitué, ou un groupe mono- ou di-(arylamino en C₆ à C₃₀) substitué ou non substitué ; ou sont liés entre des substituants adjacents pour former un cycle mono- ou polycyclique, alicyclique en (C₃ à C₃₀) ou aromatique substitué ou non substitué, dont le cycle d'atome(s) de carbone peut être remplacé par au moins un hétéroatome sélectionné parmi l'azote, l'oxygène et le soufre ;
R₅ à R₉ représentent chacun indépendamment un groupe alkyle en (C₁ à C₃₀) substitué ou non substitué, un groupe aryle en (C₆ à C₆₀) substitué ou non substitué, ou un groupe hétéroaryle à 3 à 30 chaînons substitué ou non substitué ; et
R₁₆ et R₁₇ représentent chacun indépendamment un groupe alkyle en (C₁ à C₃₀) substitué ou non substitué, un groupe cycloalkyle en (C₃ à C₃₀) substitué ou non substitué, un groupe aryle en (C₆ à C₆₀) substitué ou non substitué, ou un groupe hétéroaryle à 3 à 30 chaînons substitué ou non substitué.
